# EUROPÄISCHE PATENTSCHRIFT

(11) **EP 3 258 977 B1**
(45) Veröffentlichungstag und Bekanntmachung des Hinweises auf die Patenterteilung: **10.04.2019**
(21) Anmeldenummer: 16705110.1
(22) Anmeldetag: 16.02.2016
(51) Int. Cl.: A61L 27/50

(54) **OPHTHALMOLOGISCHE ZUSAMMENSETZUNG MIT WENIGSTENS EINER MOLEKULAREN SCHALTERVERBINDUNG**
OPHTHALMOLOGICAL COMPOSITION WITH AT LEAST ONE MOLECULAR SWITCH CONNECTION
COMPOSITION OPHTALMOLOGIQUE COMPORTANT AU MOINS UN INTERRUPTEUR MOLÉCULAIRE

(30) Priorität: 18.02.2015 DE 102015102298
(43) Veröffentlichungstag der Anmeldung: 27.12.2017
(73) Patentinhaber: Carl Zeiss Meditec AG, 07745 Jena (DE)
(72) Erfinder: WOLFSTEIN, André, 13507 Berlin (DE)
(74) Vertreter: Hofstetter, Schurack & Partner
(86) Internationale Anmeldenummer: PCT/EP2016/053215
(87) Internationale Veröffentlichungsnummer: WO 2016/131796

(56) Entgegenhaltungen:
- WO-A1-00/41650
- WO-A1-2009/124231

## Beschreibung

### Technisches Gebiet

Die Erfindung betrifft eine ophthalmologische Zusammensetzung mit wenigstens einer molekularen Schalterverbindung, welche mittels eines Reizes zwischen einem ersten und einem zweiten Zustand schaltbar ist, wobei die molekulare Schalterverbindung im ersten und im zweiten Zustand unterschiedliche Brechungsindizes besitzt. Die Erfindung betrifft weiterhin eine ophthalmologische Linse sowie ein Verfahren zum Verändern einer Brechkraft einer ophthalmologischen Linse.

### Stand der Technik

Optische Effekte von ophthalmologischen Linsen basieren auf der geometrischen Konfiguration der jeweiligen Linse. Dabei beeinflussen gewölbte oder konzentrische Oberflächenstrukturen das durchtretende Licht und verursachen aufgrund von Brechung oder Beugung unterschiedliche Lichtgeschwindigkeiten. Optische Effekte können ferner auch durch einen inhomogenen Brechungsindex innerhalb des Linsenmaterials erzeugt werden. Dazu ist es bekannt, mit Hilfe von Bestrahlung Muster in einem lichtempfindlichen Material zu erzeugen, um mit Hilfe von lichtinduzierten Reaktionen Bereiche mit unterschiedlichem Brechungsindex auszubilden. Diese Technologie kann beispielsweise verwendet werden, um den Brechungsindex einer implantierten Intraokularlinse (IOL) zu verändern und Emmetropie zu erreichen. Die individuell benötigte Brechkraft der IOL wird dabei vor der Operation für eine mutmaßliche endgültige Linsenposition im Auge berechnet. Die tatsächliche Lage der Linse kann aber von der zuvor berechneten abweichen, was zu Brechungsfehler von bis zu etwa ±1 Dioptrie führen kann. Mit Hilfe von Linsen, deren Brechkraft nachträglich einstellbar ist, kann dieses Problem ohne eine zweite Operation gelöst werden.

Eine ophthalmologische Zusammensetzung zur Herstellung einer solchen Linse ist beispielsweise aus der WO 2009/124231 A1 bekannt und umfasst Chromophore als molekularen Schalterverbindungen. Zur Veränderung der Brechkraft der ophthalmologischen Zusammensetzung bzw. der daraus hergestellten Linse werden die Schalterverbindungen bestrahlt, wodurch sie von einem nicht vernetzten ersten Zustand in einen quervernetzen Zustand oder photogebleichten zweiten Zustand übergehen, in welchem sie im Vergleich zum ersten Zustand einen unterschiedlichen Brechungsindex besitzen. Dies erlaubt eine nachträgliche Veränderung der Brechkraft der ophthalmologischen Linse.

Als nachteilig an den bekannten ophthalmologischen Zusammensetzungen ist der Umstand anzusehen, dass eine ungewollte Änderung der Brechkraft einer ophthalmologischen Linse vor, während oder nach deren Implantation in das Auge eines Patienten nur schwer verhindert werden kann. Dabei muss der Brechungsindex der Linse aber insbesondere nach dem chirurgischen Eingriff auch bei normaler Sonneneinstrahlung stabil bleiben und sich nicht unkontrolliert verändern. Vor allem photoinduzierte Radikalkettenreaktionen, die üblicherweise zum Umschalten der molekularen Schalterverbindungen vom ersten in den zweiten Zustand verwendet werden, sind aber per Definition sehr lichtempfindlich und kaum zu stoppen, sobald die Reaktion vor dem endgültigen "Lock-In", das heißt dem Fixieren des gewünschten Brechwerts, einmal gestartet ist.

### Darstellung der Erfindung

Die Aufgabe der vorliegenden Erfindung ist es, eine ophthalmologische Zusammensetzung mit wenigstens einer molekularen Schalterverbindung anzugeben, mit deren Hilfe eine unkontrollierte Änderung der Brechkraft zuverlässiger vermieden werden kann. Weitere Aufgaben der Erfindung bestehen darin, eine entsprechende ophthalmologische Linse sowie ein entsprechendes Verfahren zum Verändern einer Brechkraft einer ophthalmologischen Linse anzugeben.

Diese Aufgaben werden erfindungsgemäß durch eine ophthalmologische Zusammensetzung mit den Merkmalen des Anspruchs 1, eine ophthalmologische Linse gemäß Anspruch 8 und ein Verfahren gemäß Anspruch 10 zum Verändern einer Brechkraft einer ophthalmologischen Linse gelöst. Vorteilhafte Ausgestaltungen mit zweckmäßigen Weiterbildungen der Erfindung sind in den jeweiligen Unteransprüchen angegeben, wobei vorteilhafte Ausgestaltungen der ophthalmologischen Zusammensetzung als vorteilhafte Ausgestaltungen der ophthalmologischen Linse bzw. des Verfahrens zum Verändern einer Brechkraft einer ophthalmologischen Linse und umgekehrt anzusehen sind.

Ein erster Aspekt der Erfindung betrifft eine ophthalmologische Zusammensetzung mit wenigstens einer molekularen Schalterverbindung, wobei ein unerwünschtes Schalten der molekularen Schalterverbindung und damit eine unkontrollierte Änderung der Brechkraft erfindungsgemäß dadurch vermieden ist, dass die molekulare Schalterverbindung zumindest ein erstes Strukturelement und ein korrespondierendes zweites Strukturelement umfasst, welche nur in einem der Zustände intramolekular miteinander reagieren können. Mit anderen Worten ist es im Unterschied zum Stand der Technik vorgesehen, dass die ophthalmologische Zusammensetzung eine, zwei, drei oder mehr molekulare Schalterverbindungen umfasst, die jeweils mindestens zwei miteinander korrespondierende Strukturelemente umfassen, die nur in einem der wenigstens zwei Zustände miteinander intramolekular reagieren können. Unter korrespondierenden Strukturelementen werden im Rahmen der Erfindung grundsätzlich alle chemischen Strukturelemente und funktionellen Gruppen verstanden, die miteinander reagieren können. Besonders bevorzugt sind solche chemischen Strukturelemente und funktionellen Gruppen, die unter physiologischen Bedingungen miteinander reagieren oder mittels eines Reizes zur Reaktion gebracht werden können. Hierdurch kann der erste oder der zweite Zustand durch die intramolekulare Reaktion stabilisiert werden, wodurch ein unerwünschtes Schalten der molekularen Schalterverbindung und damit eine unerwünschte Änderung der Brechkraft zuverlässig verhindert werden. Vorzugsweise weist die molekulare Schalterverbindung hierzu eine dreidimensionale Struktur auf, die nur in einem der beiden Zustände eine für eine intramolekulare Reaktion ausreichende sterische Annäherung der miteinander korrespondierenden Strukturelemente erlaubt bzw. nur in einem der Zustände durch sterische Hinderung eine intramolekulare Reaktion der miteinander korrespondierenden Strukturelemente zumindest im Wesentlichen verunmöglicht. Alternativ oder zusätzlich kann vorgesehen sein, dass die molekulare Schalterverbindung in einem der beiden Zustände eine intramolekulare Reaktion der miteinander korrespondierenden Strukturelemente aus elektrostatischen Gründen vollständig oder zumindest im Wesentlichen verunmöglicht. Im Rahmen der vorliegenden Erfindung wird unter einer zumindest im Wesentlichen Verunmöglichung verstanden, dass unter physiologischen Bedingungen maximal 10 Mol-%, vorzugsweise maximal 2 Mol-%, insbesondere maximal 1 Mol-% der molekularen Schalterverbindung intramolekular reagiert bzw. reagieren kann. Aufgrund der intramolekularen Reaktion kann bei der erfindungsgemäßen ophthalmologische Zusammensetzung auf eine Polymerisation der molekularen Schalterverbindung vorteilhaft verzichtet werden. Dementsprechend kann die molekulare Schalterverbindung auch in sehr geringen Konzentrationen verwendet werden, da kein zusätzlicher Reaktionspartner benötigt wird. Vorzugsweise ist die intramolekulare Reaktion dabei eine nicht-radikalische Reaktion, insbesondere keine photoinduzierte Polymerisation oder Photobleichung, wodurch auch unerwünschte Nebenreaktionen beim Schalten und damit unkontrollierte Brechkraftänderungen zumindest im Wesentlichen oder vollständig verhindert werden können. Weiterhin kann grundsätzlich vorgesehen sein, dass die intramolekulare Reaktion reversibel oder irreversibel ist, wodurch der Wechsel zwischen erstem und zweitem Zustand dementsprechend reversibel oder irreversibel vorgenommen werden kann. Grundsätzlich kann vorgesehen sein, dass die molekulare Schalterverbindung im ersten Zustand einen höheren Brechungsindex als im zweiten Zustand aufweist. Alternativ kann die molekulare Schalterverbindung aber auch im zweiten Zustand einen höheren Brechungsindex aufweisen als im ersten Zustand. Durch die intramolekulare Reaktion kann wenigstens eine zusätzliche Ringstruktur gebildet werden. Ringstrukturen erhöhen normalerweise den Brechungsindex der molekularen Schalterverbindung. Dementsprechend kann durch die Zahl der über die intramolekulare Reaktion zusätzlich gebildeten Ringstrukturen eine besonders präzise Einstellung der Brechungsindexänderung vorgenommen werden. Ebenso kann vorgesehen sein, dass eine Mischung aus zwei oder mehr molekularen Schalterverbindungen vorgesehen ist, von denen manche im ersten und manche im zweiten Zustand einen höheren Brechungsindex ausweisen, wodurch eine besonders präzise Einstellbarkeit gegeben ist. Neben der wenigstens einen molekularen Schalterverbindung kann die ophthalmologische Zusammensetzung grundsätzlich ein oder mehrere polymerisierbare Monomere, ein oder mehrere UV- und/oder Violett-Absorber sowie gegebenenfalls weitere Komponenten wie beispielsweise Radikalstarter oder sonstige Initiatoren umfassen. Die ophthalmologische Zusammensetzung kann grundsätzlich zur Herstellung faltbarer oder harter ophthalmologischer Linsen ausgebildet sein und insbesondere polymerisierbare (Meth)Acryl- und/oder Silikon-Monomere als Hauptbestandteil umfassen.

In einer vorteilhaften Ausgestaltung der Erfindung ist vorgesehen, dass das erste Strukturelement wenigstens eine funktionelle Gruppe umfasst, die ausgewählt ist aus Aldehyd, Keton, Ester, Carboxygruppe, Halogenid, Carbonsäurehalogenid, Carbonsäureanhydrid und Sulfonsäure, und/oder dass das zweite Strukturelement wenigstens eine funktionelle Gruppe umfasst, die ausgewählt ist aus Amin und Alkohol. Hierdurch können die molekulare Schalterverbindung und die ophthalmologische Zusammensetzung optimal an den jeweiligen Einsatzzweck und die jeweils vorherrschenden bzw. eingestellten Bedingungen angepasst werden. Beispielsweise kann das erste Strukturelement eine Carbonsäure- bzw. Carboxygruppe sein, während das korrespondierende zweite Strukturelement eine Aminogruppe ist, die in einem der beiden Zustände der molekularen Schalterverbindung intramolekular über einen Additions-Eliminierungs-Mechanismus zu einem Imin bzw. zu einer Schiff'schen Base (Azomethin) reagieren.

Weitere Vorteile ergeben sich, indem das erste Strukturelement und das zweite Strukturelement nur in einem der Zustände kovalent, insbesondere unter intramolekularer Ringbildung, und/oder unter Ausbildung einer intramolekularen Wasserstoffbrücke und/oder unter Ausbildung einer intramolekularen Ionenbindung miteinander reagieren können. Mit anderen Worten ist es vorgesehen, dass die Stabilisierung eines der beiden Zustände der molekularen Schalterverbindung durch eine intramolekulare kovalente Ringbildung, durch Ausbildung einer oder mehrerer intramolekularer Wasserstoffbrücken zwischen dem ersten und dem zweiten Strukturelement und/oder durch intramolekulare Ionenbindung bzw. Salzbildung erfolgt. Dies erlaubt eine besonders flexible Anpassung der molekularen Schalterverbindung und der ophthalmologischen Zusammensetzung an unterschiedliche Einsatzzwecke und Verwendungsarten. Im Fall einer intramolekularen Ionenbindung liegt die molekulare Schalterverbindung in einem der Zustände als Zwitterion vor, indem eines der Strukturelemente eine positive Ladung und das andere Strukturelement eine negative Ladung trägt. Dies erlaubt eine besonders einfache, aber reversible Stabilisierung des ersten oder des zweiten Zustands, indem der pH-Wert und/oder die lonenkonzentration des Umgebungsmediums entsprechend eingestellt wird.

In einer weiteren vorteilhaften Ausgestaltung der Erfindung ist vorgesehen, dass wenigstens zwei Paare aus erstem und zweitem Strukturelement vorgesehen sind, wobei eines der Paare nur im ersten Zustand intramolekular miteinander reagieren kann und das andere Paar nur im zweiten Zustand intramolekular miteinander reagieren kann. Dies stellt eine besonders vorteilhafte Möglichkeit dar, um beide Zustände der molekularen Schalterverbindung gezielt zu stabilisieren und ein unerwünschtes Schalten zwischen erstem und zweitem Zustand besonders zuverlässig zu verhindern. Vorzugsweise reagieren das erste und das zweite Paar dabei in chemisch unterschiedlicher Weise und/oder aufgrund unterschiedlicher Reize intramolekular miteinander, wodurch ein besonders präzises Schalten der molekularen Schalterverbindung ermöglicht ist.

Weitere Vorteile ergeben sich, indem die molekulare Schalterverbindung wenigstens ein drittes Strukturelement umfasst, das beim Schalten zwischen dem ersten und dem zweiten Zustand seine Konfiguration vorzugsweise reversibel ändert. Mit anderen Worten ist erfindungsgemäß ein drittes Strukturelement vorgesehen, dass in Abhängigkeit des Zustands eine unterschiedliche räumliche Anordnung von Atomen der molekularen Schalterverbindung beziehungsweise einen unterschiedlichen räumlichen Bau der molekularen Schalterverbindung bewirkt. Dies stellt eine einfache Möglichkeit zum selektiven Einstellen des Abstands zwischen erstem und zweitem Strukturelement und damit zum Einstellen der Fähigkeit von erstem und zweitem Strukturelement zur intramolekularen Reaktion in Abhängigkeit des Zustands dar. Vorzugsweise sind das erste Strukturelement und das zweite Strukturelement derart mit dem dritten Strukturelements verbunden, dass sie beim Konfigurationswechsel des dritten Strukturelement räumlich benachbart, das heißt zur intramolekularen Reaktion befähigt oder räumlich beabstandet, das heißt unfähig zur intramolekularen Reaktion angeordnet werden.

In einer weiteren vorteilhaften Ausgestaltung der Erfindung ist vorgesehen, dass das dritte Strukturelement wenigstens eine funktionelle Gruppe umfasst, die ausgewählt ist aus Azogruppe, substituierter C=C-Doppelbindung und substituiertem Ringsystem, und/oder dass das dritte Strukturelement in Abhängigkeit des Zustands als (Z)-Isomer oder als (E)-Isomer vorliegt. Dies stellt eine besonders zuverlässige Möglichkeit dar, um das molekulare Schalterelement mit Hilfe einer Konfigurationsänderung des dritten Strukturelements zwischen dem ersten und dem zweiten Zustand zu schalten. Das dritte Strukturelement kann beispielsweise in Abhängigkeit des Zustands in einer (E)- oder (Z)-Konfiguration (bzw. in einer trans- oder cis-Konfiguration) vorliegen bzw. zwischen diesen Konfigurationen schalten.

In einer weiteren vorteilhaften Ausgestaltung der Erfindung ist die molekulare Schalterverbindung durch Temperaturänderung und/oder durch Bestrahlung mit elektromagnetischer Strahlung zwischen dem ersten und dem zweiten Zustand schaltbar. Die molekulare Schalterverbindung kann damit vorteilhafter Weise durch einen thermischen Reiz, beispielsweise durch den Unterschied zwischen Raumtemperatur und Körpertemperatur, und/oder durch elektromagnetische Strahlung, beispielsweise durch UV-Strahlung als Reiz geschaltet werden.

Weitere Vorteile ergeben sich, wenn das erste Strukturelement und das zweite Strukturelement in Abhängigkeit eines pH-Werts und/oder einer lonenkonzentration der ophthalmologischen Zusammensetzung intramolekular reagieren können. Dies stellt eine einfache Möglichkeit dar, um die molekulare Schalterverbindung durch Variierung des Umgebungs-pH-Werts und/oder der lonenkonzentration im ersten oder im zweiten Zustand zu stabilisieren.

Weitere Vorteile ergeben sich, indem die molekulare Schalterverbindung wenigstens ein viertes Strukturelement umfasst, mittels welchem die molekulare Schalterverbindung polymerisierbar ist. Hierdurch kann die molekulare Schalterverbindung vorteilhaft kovalent in ein Polymer eingebunden werden, wodurch eine Wanderung oder unerwünschte Freisetzung der molekularen Schalterverbindung besonders zuverlässig verhindert wird.

In einer weiteren vorteilhaften Ausgestaltung der Erfindung besitzt die molekulare Schalterverbindung im ersten Zustand die allgemeine Formel I

R₁-Z₁-R₂ (I),

in welcher
Z₁ (E) -N=N-, (Z) -N=N-, (E) -C=C- oder (Z) -C=C- bedeutet,
R₁ ausgewählt ist aus der Gruppe 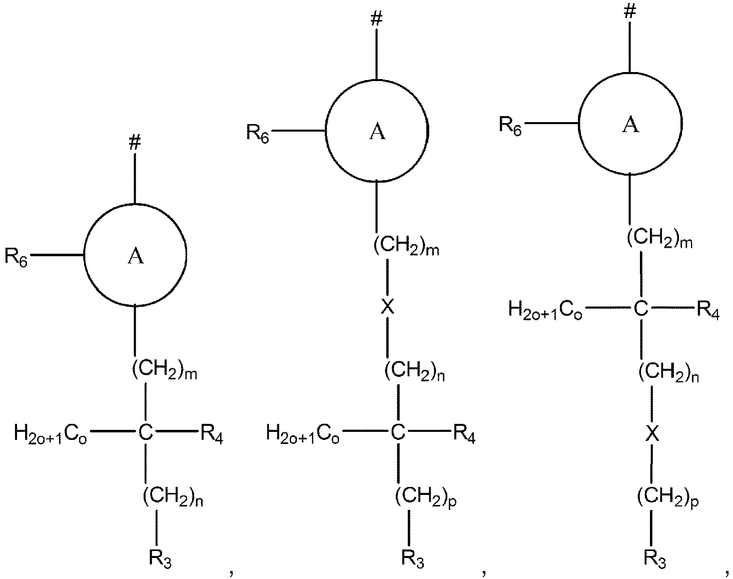 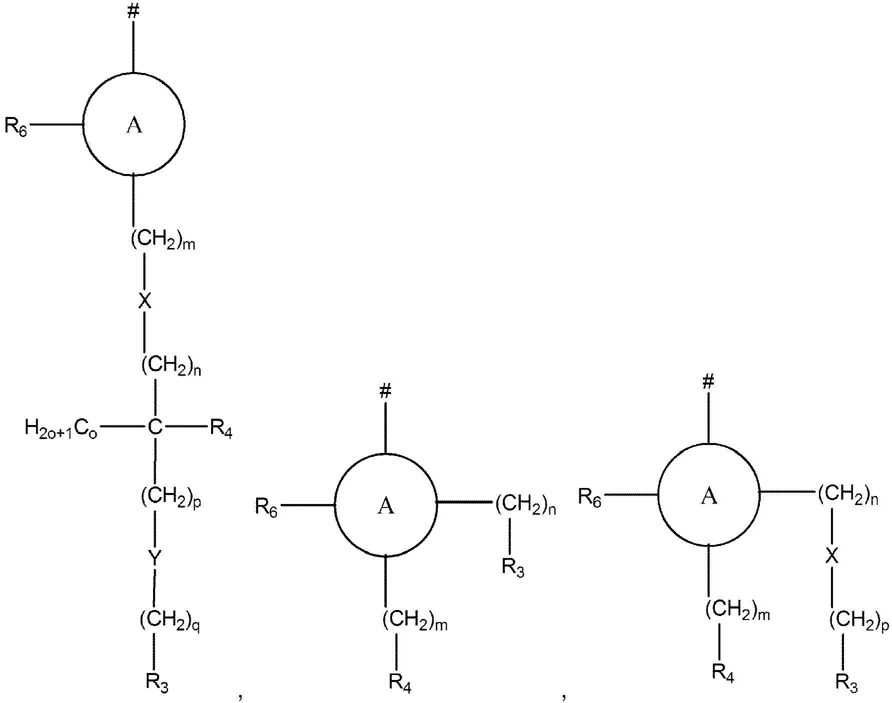 und 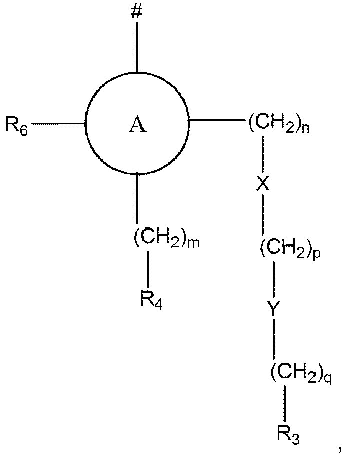 wobei
   # die Verknüpfungsstelle mit Z1 bezeichnet,
   m, n, o, p und q in R₁ jeweils unabhängig voneinander eine Ganzzahl zwischen 0 und 30 bedeuten,
   X und Y in R₁ unabhängig voneinander ausgewählt sind aus CH₂, O, S oder NR₅, wobei R₅ CᵣH₂ᵣ₊₁ mit 0≤r≤30 bedeutet,
   A ausgewählt ist aus der Gruppe 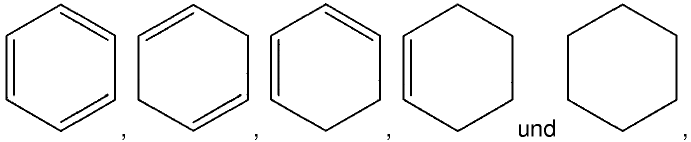
   R₂ und R₃ abhängig voneinander ausgewählt sind aus der Gruppe 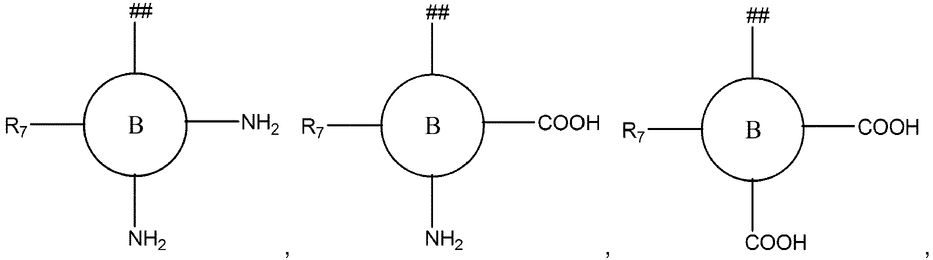 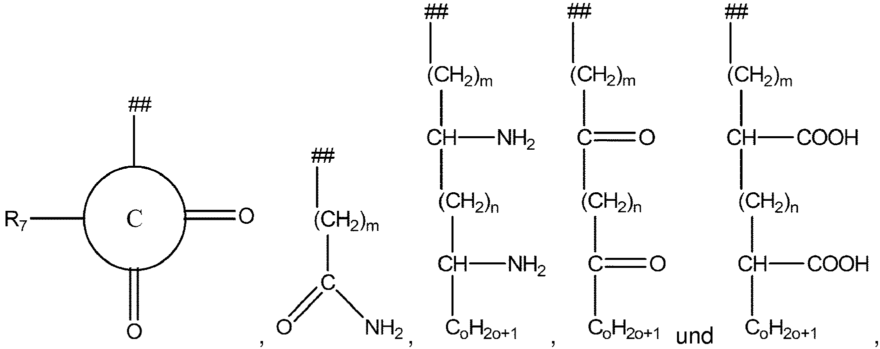 so
   dass R₂ und R₃ in einem der beiden Zustände intramolekular miteinander reagieren können, wobei
   ## im Fall von R₂ die Verknüpfungsstelle mit Z₁ und im Fall von R₃ die Verknüpfungsstelle mit R₁ bezeichnet,
   m, n und o in R₂ und R₃ jeweils unabhängig voneinander eine Ganzzahl zwischen 0 und 30 bedeuten,
   B ausgewählt ist aus der Gruppe 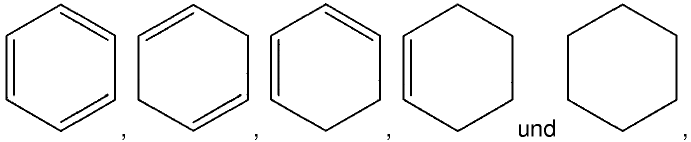
   C ausgewählt ist aus der Gruppe 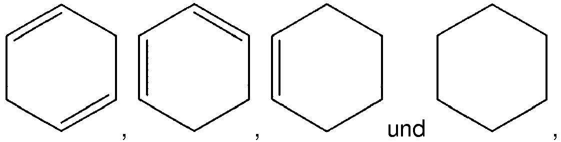
   R₄ ausgewählt ist aus der Gruppe 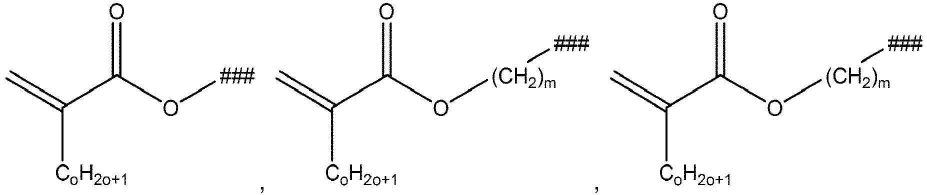 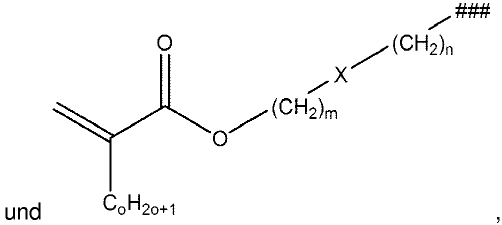 wobei
   ### die Verknüpfungsstelle mit R₁ bezeichnet,
   X in R₄ ausgewählt ist aus CH₂, O, S oder NR₅, wobei R₅ CᵣH₂ᵣ₊₁ mit 0≤r≤30 bedeutet,
   m, n und o in R₄ jeweils unabhängig voneinander eine Ganzzahl zwischen 0 und 30 bedeuten, und
   R₆ und R₇ ausgewählt sind aus 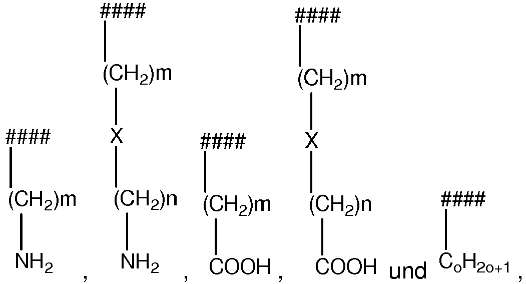 wobei
   #### im Fall von R₆ die Verknüpfungsstelle mit A und im Fall von R₇ die Verknüpfungsstelle mit B oder C bezeichnet,
   m, n und o in R₆ und R₇ jeweils unabhängig voneinander eine Ganzzahl zwischen 0 und 30 bedeuten und
   X in R₆ und R₇ unabhängig voneinander ausgewählt sind aus CH₂, O, S oder NR₅, wobei R₅ CᵣH₂ᵣ₊₁ mit 0≤r≤30 bedeutet.

Durch die allgemeine Formel I werden molekulare Schalterverbindungen bereitgestellt, die die geforderten Eigenschaften besitzen, zwischen mindestens zwei Zuständen geschaltet werden und nur in einem der Zustände intramolekular miteinander reagieren zu können, wobei sich die Brechungsindizes der Schalterverbindungen in den wenigstens zwei Zuständen unterscheiden. Hierzu werden beispielsweise R₂ und R₃, die damit als erstes und zweites Strukturelement fungieren können, derart ausgewählt, dass sie miteinander korrespondieren und in einem der Zustände intramolekular miteinander reagieren. R₂ kann dabei beispielsweise 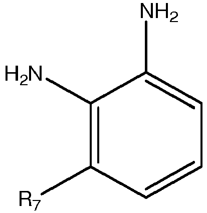 sein, während R₃ 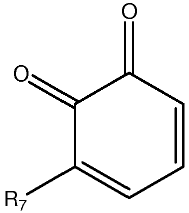 ist oder umgekehrt. Dem Fachmann sind die sich aus der angegebenen Liste an möglichen Strukturelementen ergebenden geeigneten Kombinationsmöglichkeiten bekannt. Alle Strukturelemente, die Ringsysteme umfassen können (R₁ bis R₃), können grundsätzlich über eine oder mehrere der Bindungsstellen des betreffenden Rings mit dem jeweils angrenzenden Strukturelement verknüpft sein bzw. werden. Dies ist durch die Kreise A, B und C symbolisiert. Grundsätzlich sind durch die allgemeine Formel I alle Stellungsisomere sowie alle optischen Isomere als mitoffenbart anzusehen. Ebenfalls von der allgemeinen Formel I umfasst sind Solvate, Salze sowie Solvate der Salze. Beispielsweise kann für R₂ 1,2-Diamin-3-methylphenyl (mit R₇=CH₃) als Strukturelement gewählt und über die 4-, 5- oder 6-Position des Phenylrings mit dem angrenzenden Strukturelement Z₁ verbunden werden. Dies ist im Folgenden mit Z₁ = -N=N- beispielhaft dargestellt:

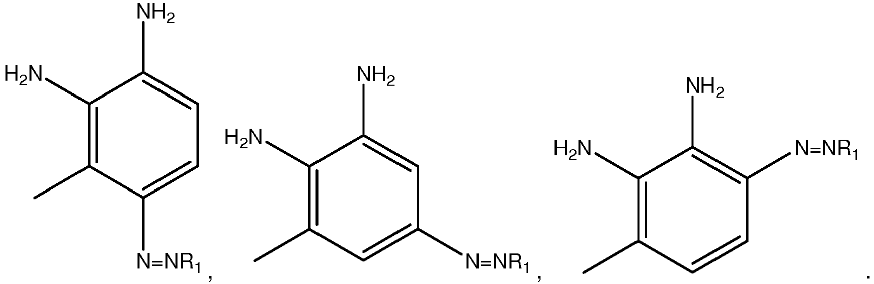

Entsprechendes gilt für die anderen Strukturelemente der allgemeinen Formel I. Das Strukturelement Z₁ der allgemeinen Formel I kann im Sinne der vorliegenden Erfindung als drittes Strukturelement fungieren, das beim Schalten zwischen dem ersten und dem zweiten Zustand seine Konfiguration vorzugsweise reversibel zwischen (E) und (Z) ändert. Die Parameter m, n, o, p, q und r können jeweils unabhängig voneinander eine Ganzzahl zwischen 0 und 30, das heißt 0, 1, 2, 3, 4, 5, 6, 7, 8, 9, 10, 11, 12, 13, 14, 15, 16, 17, 18, 19, 20, 21, 22, 23, 24, 25, 26, 27, 28, 29 oder 30 sein. Über die Strukturelemente R₆ und R₇ kann in manchen Ausführungsformen beispielsweise eine pH- und/oder lonenkonzentrations-abhängige, intramolekulare Wasserstoffbrückenbildung und/oder Ionenbindung über Zwitterionen erreicht werden, womit eine zusätzliche Möglichkeit zur Stabilisierung eines der beiden Zustände ermöglicht ist.

In einer weiteren vorteilhaften Ausgestaltung der Erfindung ist vorgesehen, dass die molekulare Schalterverbindung im ersten Zustand die Formel 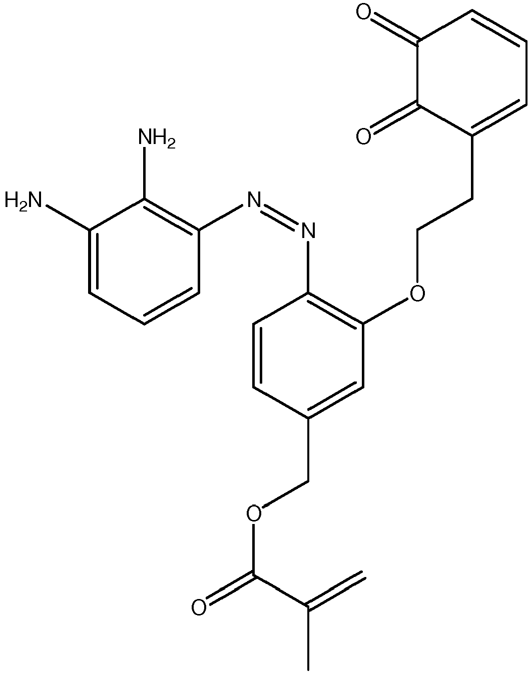 und/oder im zweiten Zustand die Formel 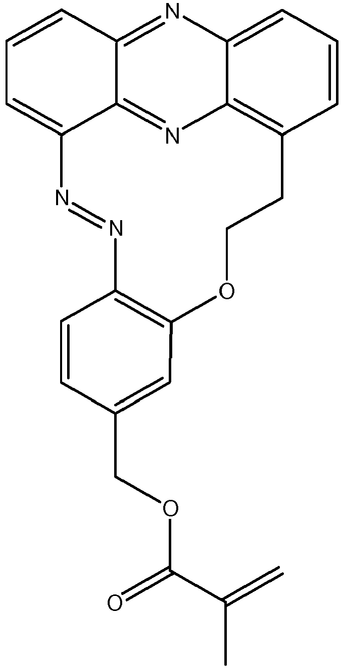 besitzt. Man erkennt, dass die molekulare Schalterverbindung im ersten Zustand eine Cyclohexadienedion-Gruppe mit zwei Ketogruppen als erste Strukturelemente sowie eine Phenyldiamin-Gruppe mit zwei Aminogruppen als zweite Strukturelemente umfasst, wobei die ersten und zweiten Strukturelemente nur im zweiten Zustand unter Ringbildung intramolekular miteinander zu einer Phenazinyl-Gruppe reagieren können. Dementsprechend weist die molekulare Schalterverbindung im ersten Zustand drei und im zweiten Zustand vier Ringstrukturen auf. Weiterhin umfasst die molekulare Schalterverbindung eine Azogruppe als drittes Strukturelement, die in Abhängigkeit des Zustands in cis- oder trans-Konfiguration vorliegt. Im Fall der thermodynamisch labileren cis-Konfiguration bzw. im ersten Zustand sind die ersten und zweiten Strukturelemente so weit voneinander beabstandet, dass sie nicht intramolekular miteinander reagieren können. In trans-Konfiguration bzw. im zweiten Zustand werden das erste und das zweite Strukturelement sterisch derart zueinander angeordnet, dass sie intramolekular unter Wasserabspaltung und Ringbildung miteinander reagieren können. Hierdurch wird der zweite Zustand, das heißt die thermodynamisch stabilere trans-Konfiguration irreversibel stabilisiert. Die molekulare Schalterverbindung kann daher beispielsweise durch vergleichsweise langwelliges Licht (>400 nm) als Reiz oder durch Wärme in die stabilere trans-Konfiguration, das heißt in den zweiten Zustand geschaltet und in diesem durch intramolekulare Ringbildung irreversibel stabilisiert werden. Schließlich umfasst die molekulare Schalterverbindung als viertes Strukturelement eine Methacrylat-Gruppe, über welche die Schalterverbindung kovalent mit weiteren Komponenten der ophthalmologischen Zusammensetzung, beispielsweise mit Acrylat- und/oder Methacrylat-Monomeren, vernetzt werden kann.

In einer weiteren vorteilhaften Ausgestaltung der Erfindung ist vorgesehen, dass die molekulare Schalterverbindung im ersten Zustand die Formel 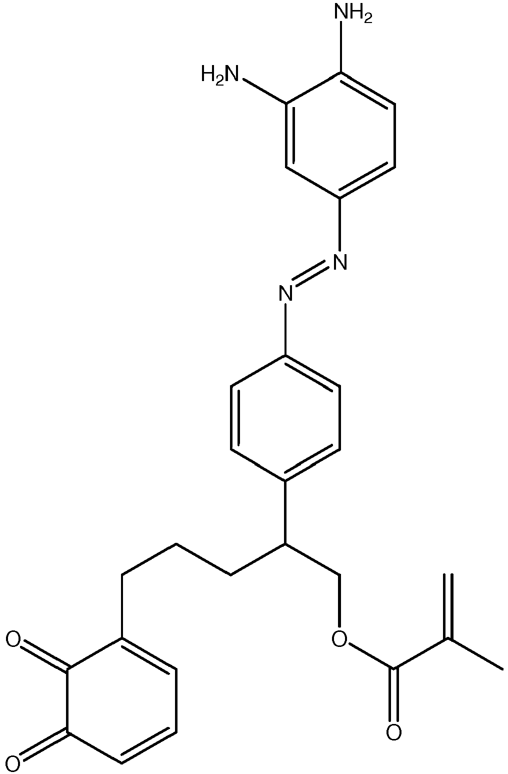 und/oder im zweiten Zustand die Formel 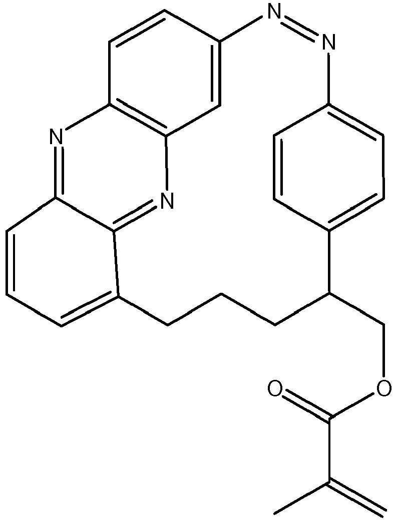 besitzt. Auch im vorliegenden Fall umfasst die molekulare Schalterverbindung im ersten Zustand eine Cyclohexadienedion-Gruppe mit zwei Ketogruppen als erste Strukturelemente sowie eine Phenyldiamin-Gruppe mit zwei Aminogruppen als zweite Strukturelemente umfasst, die im zweiten Zustand unter Ringbildung intramolekular zu einer Phenazinyl-Gruppe reagieren, um den zweiten Zustand zu stabilisieren. Weiterhin umfasst die molekulare Schalterverbindung eine Azogruppe als drittes Strukturelement, die in Abhängigkeit des Zustands in trans- oder cis-Konfiguration vorliegt. Im Fall der thermodynamisch stabileren trans-Konfiguration bzw. im ersten Zustand sind die ersten und zweiten Strukturelemente so weit voneinander beabstandet, dass sie nicht intramolekular miteinander reagieren können. In cis-Konfiguration bzw. im zweiten Zustand werden das erste und das zweite Strukturelement derart zueinander angeordnet, dass sie intramolekular unter Wasserabspaltung und Ringbildung reagieren können. Hierdurch kann der zweite, thermodynamisch labilere Zustand dauerhaft stabilisiert werden. Die molekulare Schalterverbindung kann daher beispielsweise durch vergleichsweise kurzwelliges Licht (ca. 300-400 nm) als Reiz in die labilere cis-Konfiguration, das heißt in den zweiten Zustand geschaltet und in diesem durch intramolekulare Ringbildung irreversibel stabilisiert werden. Schließlich umfasst die molekulare Schalterverbindung als viertes Strukturelement eine Methacrylat-Gruppe, über welche die Schalterverbindung kovalent in ein Polymer, beispielsweise ein Acrylat bzw. Methacrylat eingebunden werden kann.

In einer weiteren vorteilhaften Ausgestaltung der Erfindung ist vorgesehen, dass die molekulare Schalterverbindung im ersten Zustand die Formel 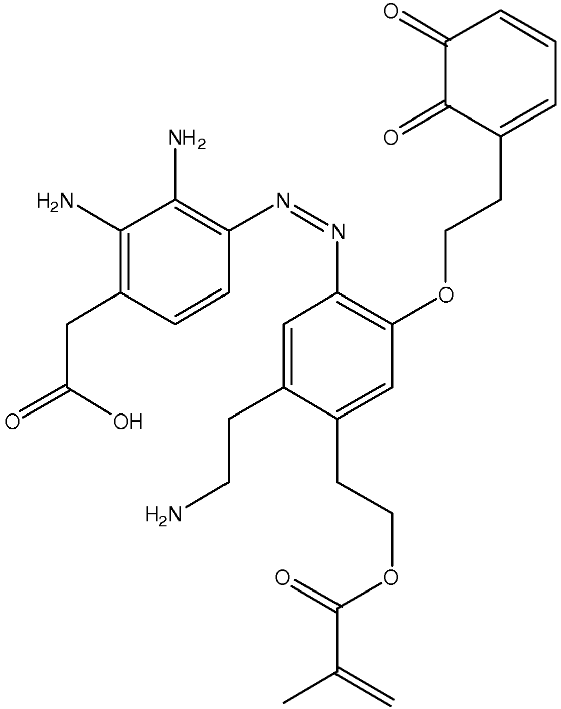 und/oder im zweiten Zustand die Formel 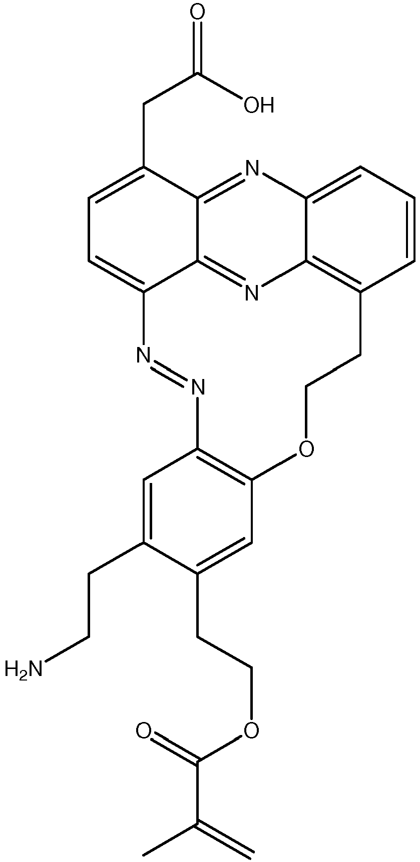 besitzt. Die molekulare Schalterverbindung weist im ersten Zustand zwei unterschiedliche Paare aus erstem und zweitem Strukturelement auf. Das erste Paar Strukturelemente umfasst erneut zwei Ketogruppen als erste Strukturelemente sowie zwei Aminogruppen als zweite Strukturelemente. Als zweites Paar Strukturelemente umfasst die molekulare Schalterverbindung eine Carboxy-Gruppe bzw. einen Acetat-Rest an der Phenyldiamin-Gruppe als erstes Strukturelement. Als korrespondierendes zweites Strukturelement des zweiten Paars ist eine weitere Aminogruppe bzw. ein Ethylamin-Rest vorgesehen. Weiterhin umfasst die molekulare Schalterverbindung eine Azogruppe als drittes Strukturelement, die in Abhängigkeit des Zustands in cis- oder trans-Konfiguration vorliegt. Man erkennt, dass das zweite Paar aus erstem und zweitem Strukturelement im ersten Zustand, das heißt in cis-Konfiguration, ein Zwitterion mit einer positiven Ladung an der Amino- bzw. AmmoniumGruppe und mit einer negativen Ladung an der benachbarten Carboxy- bzw. CarboxylatGruppe bilden und damit den ersten Zustand durch intramolekulare Salzbildung stabilisieren kann. Im zweiten Zustand ist diese intramolekulare Ionenbindung aufgrund des großen Abstands der beiden Strukturelemente des zweiten Paars nicht möglich. Im zweiten Zustand können aber erneut die beiden ersten und zweiten Strukturelemente des ersten Paars unter Ringbildung kovalent unter Ringbildung zu einer Phenazinyl-Gruppe reagieren und damit den zweiten Zustand über einen alternativen Mechanismus stabilisieren. Das bedeutet, dass der erste Zustand durch Einstellung eines etwa neutralen pH-Werts und Bildung eines vicinalen Zwitterions stabilisiert werden kann, da der pKa-Wert der Carboxy-Gruppe etwa 9,73 (Phenylessigsäure) beträgt, während der pKa-Wert der Aminogruppe etwa 4,31 (Phenylethylamin) beträgt. Bei sauren pH-Werten <7 wird das Carboxylat-Ion protoniert und die Stabilisierung des ersten Zustands aufgehoben. Unter sauren Bedingungen ist der erste Zustand (cis-Konfiguration) instabiler und kann relativ einfach zum zweiten Zustand (trans-Konfiguration) umlagern bzw. umgelagert werden. Im zweiten Zustand können dann die beiden ersten und zweiten Strukturelemente des ersten Paars miteinander reagieren und den zweiten Zustand durch intramolekulare Ringbildung, beispielsweise aufgrund einer photoinduzierten oder thermisch induzierten Reaktion, irreversibel stabilisieren. Alternativ kann die molekulare Schalterverbindung durch erneutes Einstellen eines neutralen pH-Werts wieder in den ersten Zustand zurückgeschaltet und in diesem durch intramolekulare Salzbildung wieder stabilisiert werden. Alternativ oder zusätzlich zur Einstellung eines sauren pH-Werts kann der erste Zustand auch durch Einstellung einer hohen lonenstärke bzw. einer hohen lonenkonzentration destabilisiert werden. Beispielsweise kann das Zwitterion durch Zugabe von NaCl, das heißt durch Bereitstellung entsprechender Gegenionen destabilisiert und in den zweiten Zustand umgelagert werden, da die intramolekulare Salzbildung zerstört wird. Schließlich umfasst die molekulare Schalterverbindung als viertes Strukturelement eine Methacrylat-Gruppe, über welche die Schalterverbindung kovalent mit weiteren Komponenten der ophthalmologischen Zusammensetzung, beispielsweise mit Acrylat- und/oder Methacrylat-Monomeren, vernetzt werden kann.

In einer weiteren vorteilhaften Ausgestaltung der Erfindung ist vorgesehen, dass die molekulare Schalterverbindung im ersten Zustand die Formel 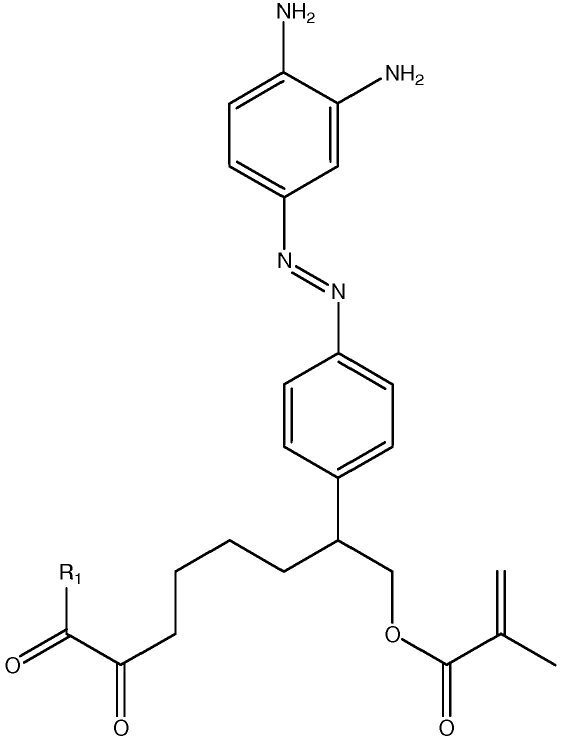
mit R₁=Wasserstoff oder CₙH₂ₙ₊₁ mit 1 ≤ n ≤ 30
und/oder im zweiten Zustand die Formel 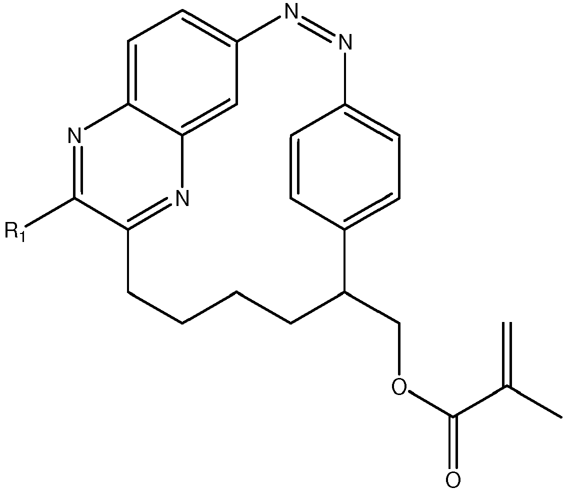 besitzt. Man erkennt, dass die molekulare Schalterverbindung im Unterschied zu den vorhergehenden Ausführungsbeispielen im ersten Zustand nur zwei und im zweiten Zustand nur drei Ringstrukturen aufweist.

In einer weiteren vorteilhaften Ausgestaltung der Erfindung ist vorgesehen, dass die molekulare Schalterverbindung im ersten Zustand die Formel 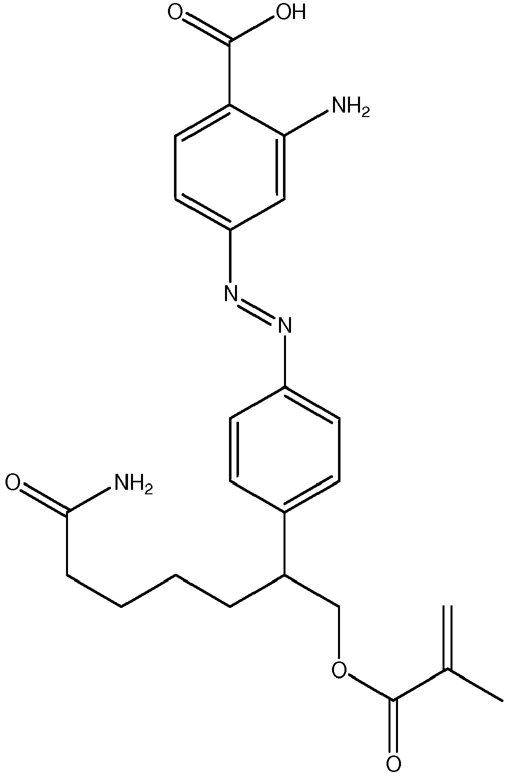 und/oder im zweiten Zustand die Formel 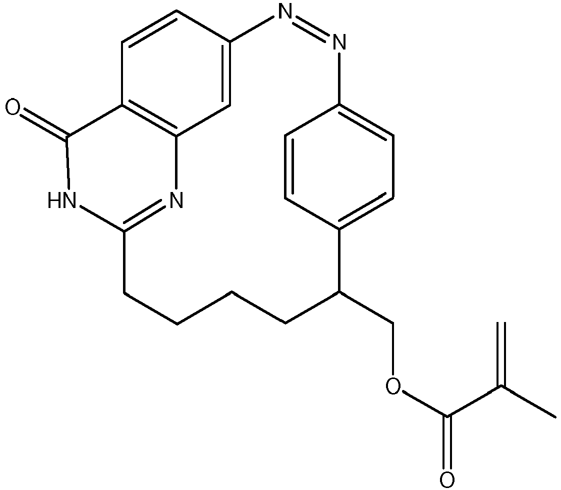 besitzt. Auch in diesem Fall weist die molekulare Schalterverbindung im ersten Zustand zwei und im zweiten Zustand drei Ringstrukturen auf.

In einer weiteren vorteilhaften Ausgestaltung der Erfindung ist vorgesehen, dass die molekulare Schalterverbindung im ersten Zustand die Formel 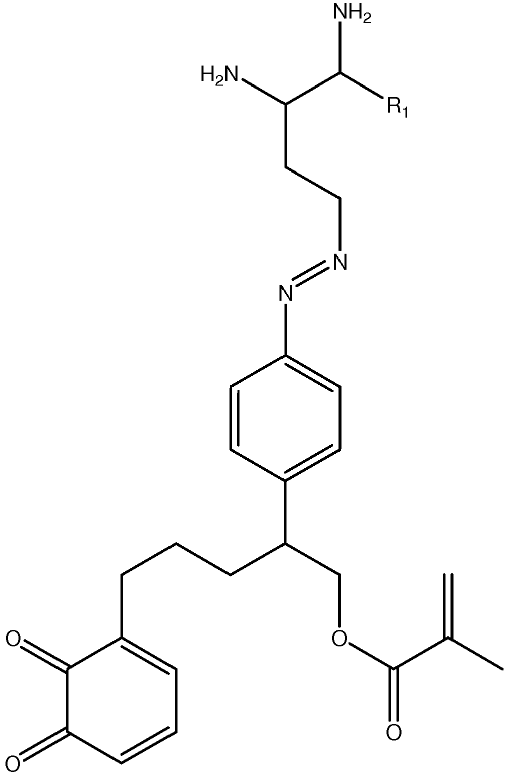 und/oder im zweiten Zustand die Formel 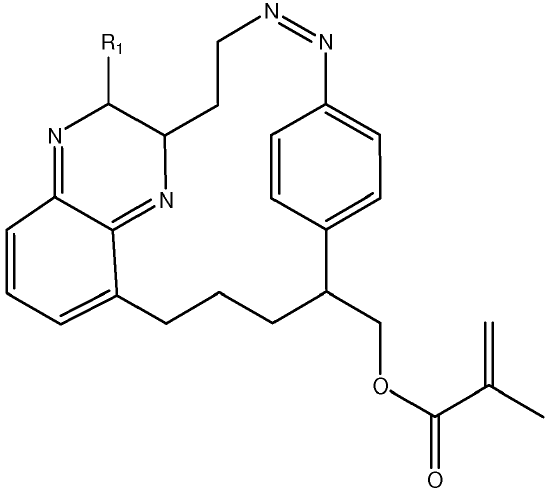 besitzt. Auch in diesem Fall weist die molekulare Schalterverbindung im ersten Zustand zwei und im zweiten Zustand drei Ringstrukturen auf.

In einer weiteren vorteilhaften Ausgestaltung der Erfindung ist vorgesehen, dass die molekulare Schalterverbindung im ersten Zustand die Formel 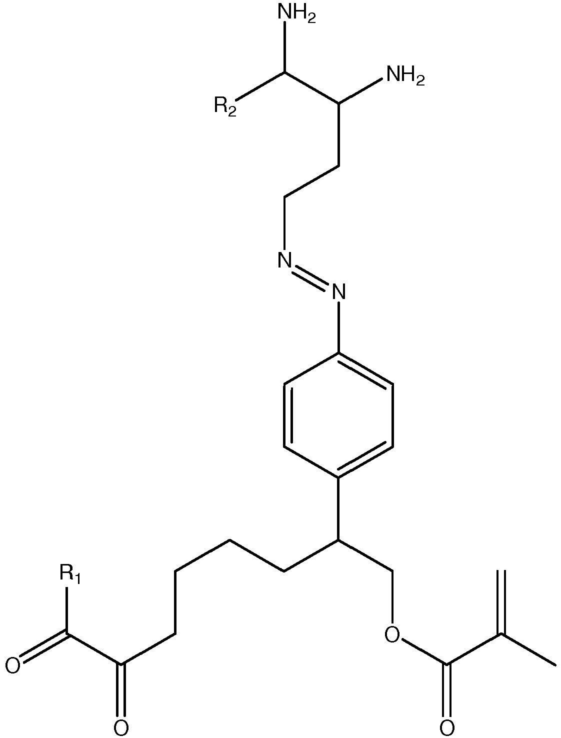 und/oder im zweiten Zustand die Formel 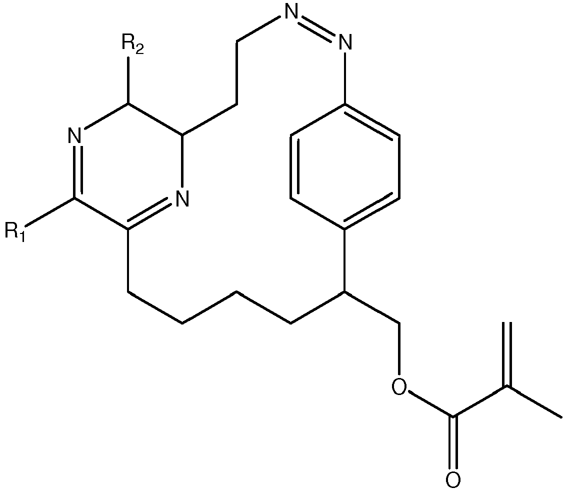 besitzt. Man erkennt, dass die molekulare Schalterverbindung im Unterschied zu den vorhergehenden Ausführungsbeispielen im ersten Zustand nur eine und im zweiten Zustand nur zwei Ringstrukturen aufweist.

In einer weiteren vorteilhaften Ausgestaltung der Erfindung umfasst die ophthalmologische Zusammensetzung zumindest HEMA (2-Hydroxyethylmethacrylat) und eine oder mehrere molekulare Schalterverbindung(en). Zusätzlich kann vorgesehen sein, dass die ophthalmologische Zusammensetzung eine oder mehrere Komponenten aus der Gruppe EA (Ethylacrylat), EMA (Ethylmethacrylat), TFEMA (Trifluorethylmethacrylat), MMA (Methylmethacrylat), EOEMA (2-Ethoxyethylmethacrylat), EGDMA (Ethylenglykoldimethacrylat), Radikalstarter, UV-Absorber und Violett-Absorber umfasst oder aus den betreffenden Komponenten besteht. Eine erfindungsgemäße ophthalmologische Zusammensetzung kann beispielsweise aus

| | |
|---|---|
| HEMA (2-Hydroxyethylmethacrylat): | 60-90 % |
| Molekulare Schalterverbindung: | 0,1-30 % |
| EOEMA (2-Ethoxyethylmethacrylat): | 0-30 % |
| EGDMA (Ethylenglykoldimethacrylat): | 0,25-1,5 % |
| Radikalstarter | 0,01-0,1 % |
| UV-Absorber: | 0-1,0 % |
| Violett-Absorber: | 0-0,16 % |

bestehen. Alle Prozentangaben sind im Rahmen der vorliegenden Erfindung als Massenprozente zu verstehen, sofern nichts anderes angegeben ist. Unter 60-90 % HEMA sind insbesondere 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 % oder 90 % HEMA zu verstehen. Unter 0-30 % EOEMA sind insbesondere 0 %, 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 % oder 30 % EOEMA zu verstehen. Unter 0,1-30 % molekulare Schalterverbindung sind insbesondere 0,1 %, 0,2 %, 0,5 %, 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 % oder 30 % molekulare Schalterverbindung zu verstehen. Unter 0,25-1,5 % EGDMA (Ethylenglykoldimethacrylat) sind insbesondere 0,25 %, 0,3 %, 0,4 %, 0,5 %, 0,6 %, 0,7 %, 0,8 %, 0,9 %, 1,0 %, 1,1 %, 1,2 %, 1,3 %, 1,4 % oder 1,5 % EGDMA zu verstehen. Unter 0,01-0,1 % Radikalstarter sind insbesondere 0,01 %, 0,02 %, 0,03 %, 0,04 %, 0,05 %, 0,06 %, 0,07 %, 0,08 %, 0,09 % oder 0,10 % Radikalstarter zu verstehen. Unter 0-1,0 % UV-Absorber sind insbesondere 0 %, 0,01 %, 0,02 %, 0,03 %, 0,04 %, 0,05 %, 0,06 %, 0,07 %, 0,08 %, 0,09 % oder 0,10 % UV-Absorber zu verstehen. Unter 0-0,16 % Violett-Absorber sind insbesondere 0 %, 0,01 %, 0,02 %, 0,03 %, 0,04 %, 0,05 %, 0,06 %, 0,07 %, 0,08 %, 0,09 %, 0,10 %, 0,11 %, 0,12 %, 0,13 %, 0,14 %, 0,15 % oder 0,16 % Violett-Absorber zu verstehen. Durch Angabe von Bereichen sind im Rahmen der vorliegenden Erfindung grundsätzlich auch alle Zwischenwerte als mitoffenbart anzusehen. Es versteht sich, dass die Anteile der einzelnen Komponenten der ophthalmologischen Zusammensetzung so zu wählen sind, dass sie sich immer und ausschließlich zu 100 % ergänzen. Durch Variierung des Anteils an molekularer Schalterverbindung am Gesamtgewicht der ophthalmologischen Zusammensetzung kann deren Brechungsindex in weiten oder engen Bereichen nachträglich nicht-invasiv, das heißt beispielsweise nach der Implantation einer IOL in ein Patientenauge, eingestellt werden. Es kann vorgesehen sein, dass das Verhältnis EOEMA zu molekularer Schalterverbindung variiert wird, um den Brechungsindex der ophthalmologischen Zusammensetzung um bis zu 0,14 zu erhöhen oder zu erniedrigen. Ab einem Massenverhältnis von etwa 30 % der molekularen Schalterverbindung an der Gesamtmasse der ophthalmologischen Zusammensetzung können durch Schalten von zumindest annähernd 100 % der molekularen Schalterverbindungsmoleküle Änderung von bis zu 10 Dioptrien (D) bei einer aus der ophthalmologischen Zusammensetzung gefertigten Linse erzielt werden. Werden nur 50 % der vorhandenen Schalterverbindung geschaltet, ist eine entsprechende Änderung von etwa 5,4 D möglich. Bei der postoperativen Einstellung einer IOL sind insbesondere nachträglich einstellbare Differenzen von 0,5 D bis 1 D therapeutisch sinnvoll, so dass in der Regel ein Anteil von etwa 10 % bis 20 % an molekularer Schalterverbindung bezogen auf die Gesamtmasse der ophthalmologischen Zusammensetzung ausreicht.

Ein zweiter Aspekt der Erfindung betrifft eine ophthalmologische Linse, wobei eine unkontrollierte Änderung der Brechkraft zuverlässiger dadurch verhindert werden kann, dass diese eine ophthalmologische Zusammensetzung nach einem der vorhergehenden Ausführungsbeispiele umfasst. Die sich hieraus ergebenden Merkmale und deren Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

In einer vorteilhaften Ausgestaltung der Erfindung ist vorgesehen, dass die ophthalmologische Linse als Intraokularlinse oder als Kontaktlinse oder als Brillenglas ausgebildet ist.

Weitere Vorteile ergeben sich, wenn die ophthalmologische Linse eine Beschichtung aufweist, welche die ophthalmologische Zusammensetzung umfasst oder aus der ophthalmologischen Zusammensetzung besteht. Hierdurch ist eine besonders hohe Freiheit bei der Materialauswahl, dem Aufbau und der Geometrie der ophthalmologische Linse ermöglicht. Zusätzlich können auf diese Weise diffraktive Strukturen an der Oberfläche der ophthalmologischen Linse erzeugt werden. Die Schichtdicke der Beschichtung beträgt vorzugsweise mindestens 1 µm, insbesondere mindestens 10 µm, um eine ausreichende Beeinflussung des Brechungsindex der ophthalmologischen Linse mit Hilfe der wenigstens einen molekularen Schalterverbindung zu ermöglichen. Grundsätzlich kann die Schichtdicke der Beschichtung aber bedarfsweise eingestellt werden und beispielsweise 0,1 µm, 0,5 µm, 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm oder mehr betragen. Ebenso können zwei oder mehr Schichten vorgesehen sein, die die gleiche oder eine unterschiedliche ophthalmologische Zusammensetzung aufweisen.

Ein dritter Aspekt der Erfindung betrifft ein Verfahren zum Verändern einer Brechkraft einer ophthalmologischen Linse, wobei die ophthalmologische Linse eine ophthalmologische Zusammensetzung gemäß dem ersten Erfindungsaspekt umfasst und/oder gemäß dem zweiten Erfindungsaspekt ausgebildet ist. Erfindungsgemäß kann eine unkontrollierte Änderung der Brechkraft zuverlässiger dadurch verhindert werden, dass die wenigstens eine molekularen Schalterverbindung mittels eines Reizes zwischen einem ersten und einem zweiten Zustand geschaltet wird, wodurch sich die Brechkraft der ophthalmologischen Linse ändert, wobei das zumindest eine erste Strukturelement und das korrespondierende zweite Strukturelement nur in einem der Zustände intramolekular miteinander zur Reaktion gebracht werden. Die sich hieraus ergebenden Merkmale und deren Vorteile sind den vorhergehenden Beschreibungen des ersten und zweiten Erfindungsaspekts zu entnehmen und gelten entsprechend für das erfindungsgemäße Verfahren.

Das erfindungsgemäße Verfahren zum Verändern der Brechkraft der ophthalmologischen Linse kann beispielsweise während bzw. im Anschluss an eine Katarakt-Operation durchgeführt werden. Sobald die Intraokularlinse an der gewünschten Position im Kapselsack angeordnet und gegebenenfalls entfalten und fixiert wurde, kann die notwendige Brechkraft bestimmt und mit Hilfe der molekularen Schalterverbindung gegebenenfalls nachjustiert werden. Hierzu wird die molekulare Schalterverbindung bedarfsweise zwischen dem ersten und dem zweiten Zustand geschaltet und im jeweils gewünschten Zustand stabilisiert.

Weitere Merkmale der Erfindung ergeben sich aus den Ansprüchen und den Ausführungsbeispielen. Die vorstehend in der Beschreibung genannten Merkmale und Merkmalskombinationen, sowie die nachfolgend in den Ausführungsbeispielen genannten und/oder alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Ausführungsbeispielen nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen.

### Bevorzugte Ausführung der Erfindung

### Ausführungsbeispiel 1

Es wird eine molekulare Schalterverbindung bereitgestellt, welche mittels eines Reizes zwischen einem ersten und einem zweiten Zustand schaltbar ist, wobei die molekulare Schalterverbindung im ersten und im zweiten Zustand unterschiedliche Brechungsindizes besitzt. Im ersten Zustand besitzt die molekulare Schalterverbindung die Formel 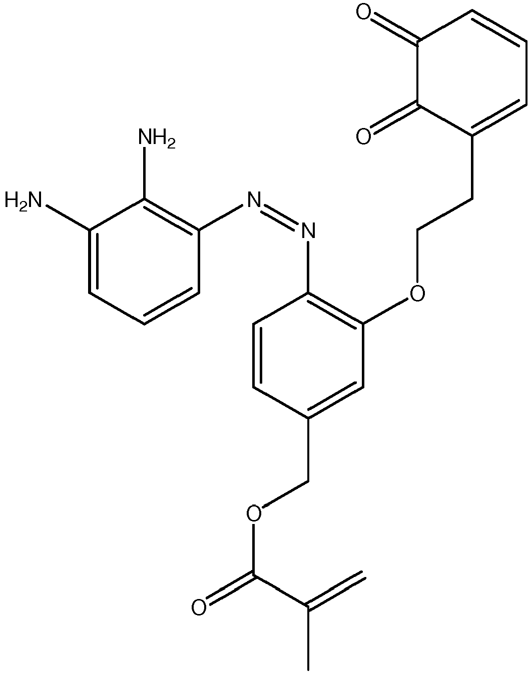 und im zweiten Zustand die Formel

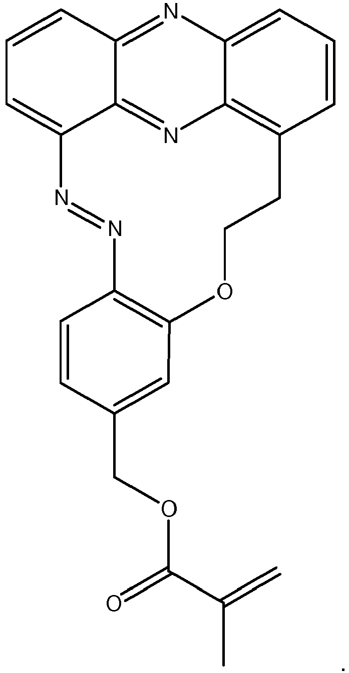

Man erkennt, dass die molekulare Schalterverbindung eine substituierte Azophenyl-Gruppe als drittes Strukturelement umfasst. Anstelle einer Azophenyl-Gruppe kann grundsätzlich auch eine Diphenylethen-Gruppe oder Ähnliches vorgesehen sein. Einer der Phenylreste der Azophenyl-Gruppe ist über eine Ethoxy-Gruppe mit einer Cyclohexadienedion-Gruppe verbunden, die zwei Ketogruppen als zwei erste Strukturelemente aufweist. Anstelle einer Ethoxy-Gruppe können auch andere Spacer wie beispielsweise C1, C2, C3, C4, oder C5-AlkylReste vorgesehen sein. Über den Spacer kann der Abstand zwischen erstem und zweitem Strukturelement eingestellt werden, um eine intramolekulare Reaktion in einem der beiden Zustände zu ermöglichen bzw. im anderen Zustand zu verunmöglichen.

Der andere Phenylrest der Azophenyl-Gruppe ist als Phenyldiamin-Gruppe mit zwei Aminogruppen als zwei zweite Strukturelemente ausgebildet. Das dritte Strukturelement liegt in Abhängigkeit des Zustands in cis- oder trans-Konfiguration vor. Die ersten und zweiten Strukturelemente können dabei nur im zweiten Zustand unter Wasserabspaltung und Ringbildung intramolekular miteinander zu einer Phenazinyl-Gruppe reagieren:

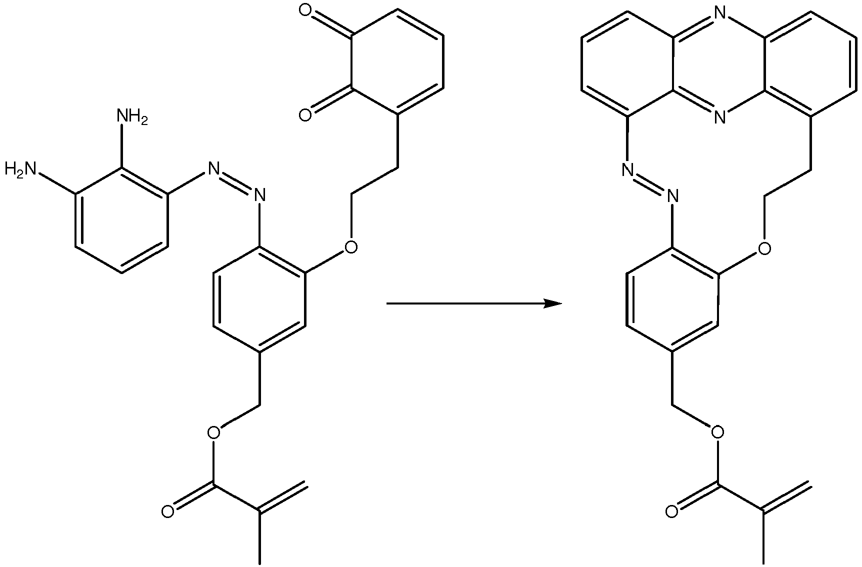

Das Schalten vom ersten in den zweiten Zustand kann durch Bestrahlung mit elektromagnetischer Strahlung im sichtbaren Wellenlängenbereich (>400 nm) und/oder durch Erwärmen erfolgen.

Im Fall der thermodynamisch labileren cis-Konfiguration bzw. im ersten Zustand sind die ersten und zweiten Strukturelemente so weit voneinander beabstandet, dass sie nicht intramolekular miteinander reagieren können. In trans-Konfiguration bzw. im zweiten Zustand werden das erste und das zweite Strukturelement sterisch derart zueinander angeordnet, dass sie intramolekular unter Wasserabspaltung und Ringbildung miteinander reagieren können. Hierdurch wird der zweite Zustand, das heißt die thermodynamisch stabilere trans-Konfiguration irreversibel stabilisiert.

Schließlich umfasst die molekulare Schalterverbindung als viertes Strukturelement eine grundsätzlich optionale Methacrylat-Gruppe, über welche die Schalterverbindung kovalent mit weiteren Komponenten der ophthalmologischen Zusammensetzung, beispielsweise mit Acrylat- und/oder Methacrylat-Monomeren, vernetzt werden kann. Anstelle einer (Meth)acrylat-Gruppe können auch andere polymerisierbare Gruppen, beispielsweise eine Vinyl-Gruppe, vorgesehen sein, die ebenfalls über einen individuell einstellbaren Spacer mit einem der Phenylringe der Azophenyl-Gruppe verknüpft sein kann.

### Ausführungsbeispiel 2

In einem weiteren Ausführungsbeispiel der Erfindung ist vorgesehen, dass die molekulare Schalterverbindung im ersten Zustand die Formel 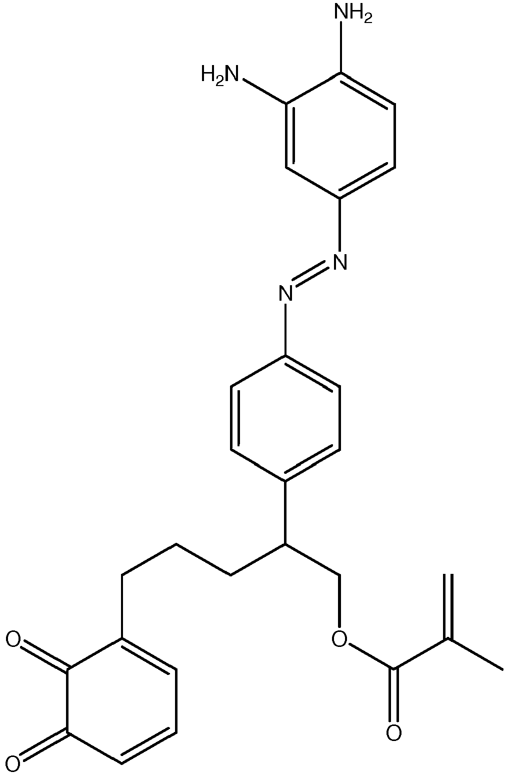 und im zweiten Zustand die Formel 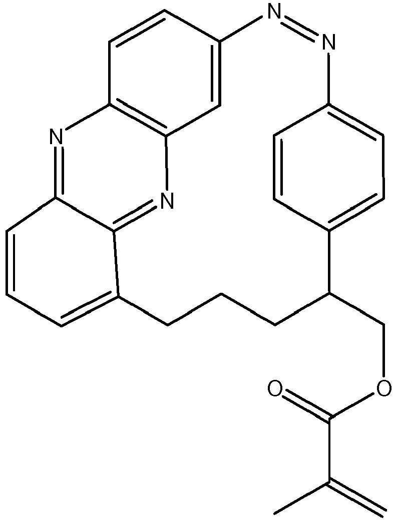 besitzt. Auch im vorliegenden Fall umfasst die molekulare Schalterverbindung im ersten Zustand eine Cyclohexadiendion-Gruppe mit zwei Ketogruppen als erste Strukturelemente sowie eine Phenyldiamin-Gruppe mit zwei Aminogruppen als zweite Strukturelemente umfasst, die im zweiten Zustand unter Ringbildung intramolekular zu einer Phenazinyl-Gruppe reagieren, um den zweiten Zustand zu stabilisieren. Weiterhin umfasst die molekulare Schalterverbindung eine Azo- bzw. Azophenyl-Gruppe als drittes Strukturelement, die in Abhängigkeit des Zustands in trans- oder cis-Konfiguration vorliegt. Im Fall der thermodynamisch stabileren trans-Konfiguration bzw. im ersten Zustand sind die ersten und zweiten Strukturelemente so weit voneinander beabstandet, dass sie nicht intramolekular miteinander reagieren können. In cis-Konfiguration bzw. im zweiten Zustand werden das erste und das zweite Strukturelement derart zueinander angeordnet, dass sie intramolekular unter Wasserabspaltung und Ringbildung reagieren können:

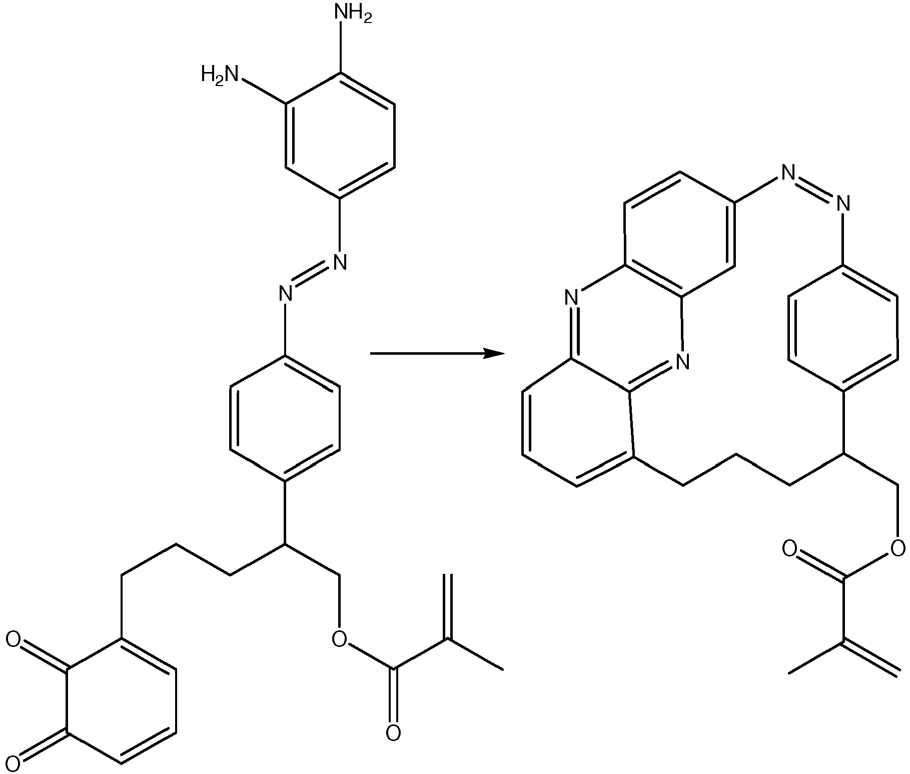

Hierdurch kann der zweite, thermodynamisch labilere Zustand dauerhaft stabilisiert werden. Die molekulare Schalterverbindung kann daher beispielsweise durch vergleichsweise kurzwelliges Licht (ca. 300-400 nm) als Reiz in die labilere cis-Konfiguration, das heißt in den zweiten Zustand geschaltet und in diesem durch intramolekulare Ringbildung irreversibel stabilisiert werden. Schließlich umfasst die molekulare Schalterverbindung als viertes Strukturelement eine grundsätzlich optionale Methacrylat-Gruppe, über welche die Schalterverbindung kovalent in ein Polymer, beispielsweise ein Acrylat bzw. Methacrylat eingebunden werden kann.

### Ausführungsbeispiel 3

In einem weiteren Ausführungsbeispiel der Erfindung weist die molekulare Schalterverbindung im ersten Zustand die Formel 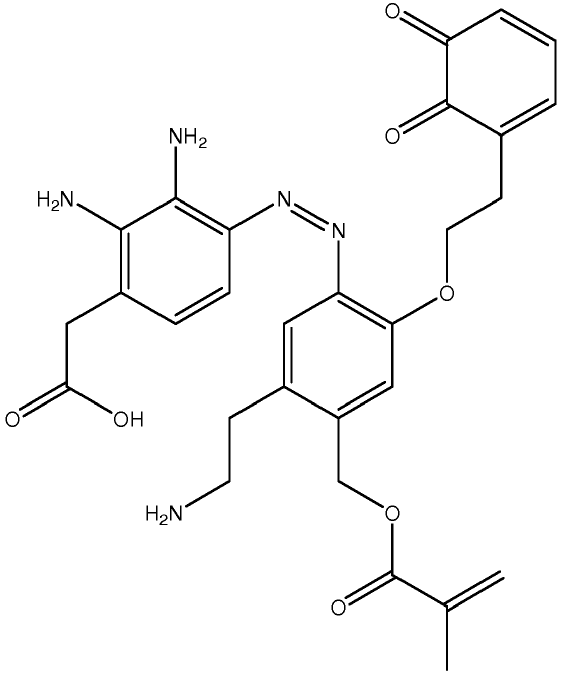 und im zweiten Zustand die Formel 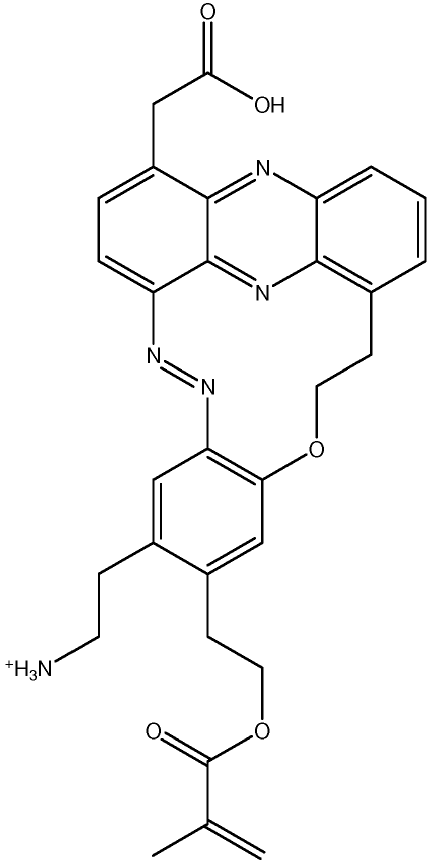 auf. Die molekulare Schalterverbindung weist im ersten Zustand zwei unterschiedliche Paare aus erstem und zweitem Strukturelement auf. Das erste Paar Strukturelemente umfasst erneut zwei Ketogruppen als erste Strukturelemente sowie zwei Aminogruppen als zweite Strukturelemente. Als zweites Paar Strukturelemente umfasst die molekulare Schalterverbindung eine Carboxy-Gruppe bzw. einen Acetat-Rest an der Phenyldiamin-Gruppe als erstes Strukturelement. Als korrespondierendes zweites Strukturelement des zweiten Paars ist eine weitere Aminogruppe bzw. ein Ethylamin-Rest vorgesehen. Weiterhin umfasst die molekulare Schalterverbindung eine Azogruppe als drittes Strukturelement, die in Abhängigkeit des Zustands wieder in cis- oder trans-Konfiguration vorliegt. Man erkennt, dass das zweite Paar aus erstem und zweitem Strukturelement im ersten Zustand, das heißt in cis-Konfiguration, ein Zwitterion mit einer positiven Ladung an der Amino- bzw. AmmoniumGruppe und mit einer negativen Ladung an der benachbarten Carboxy- bzw. CarboxylatGruppe bilden und damit den ersten Zustand durch intramolekulare Salzbildung stabilisieren kann:

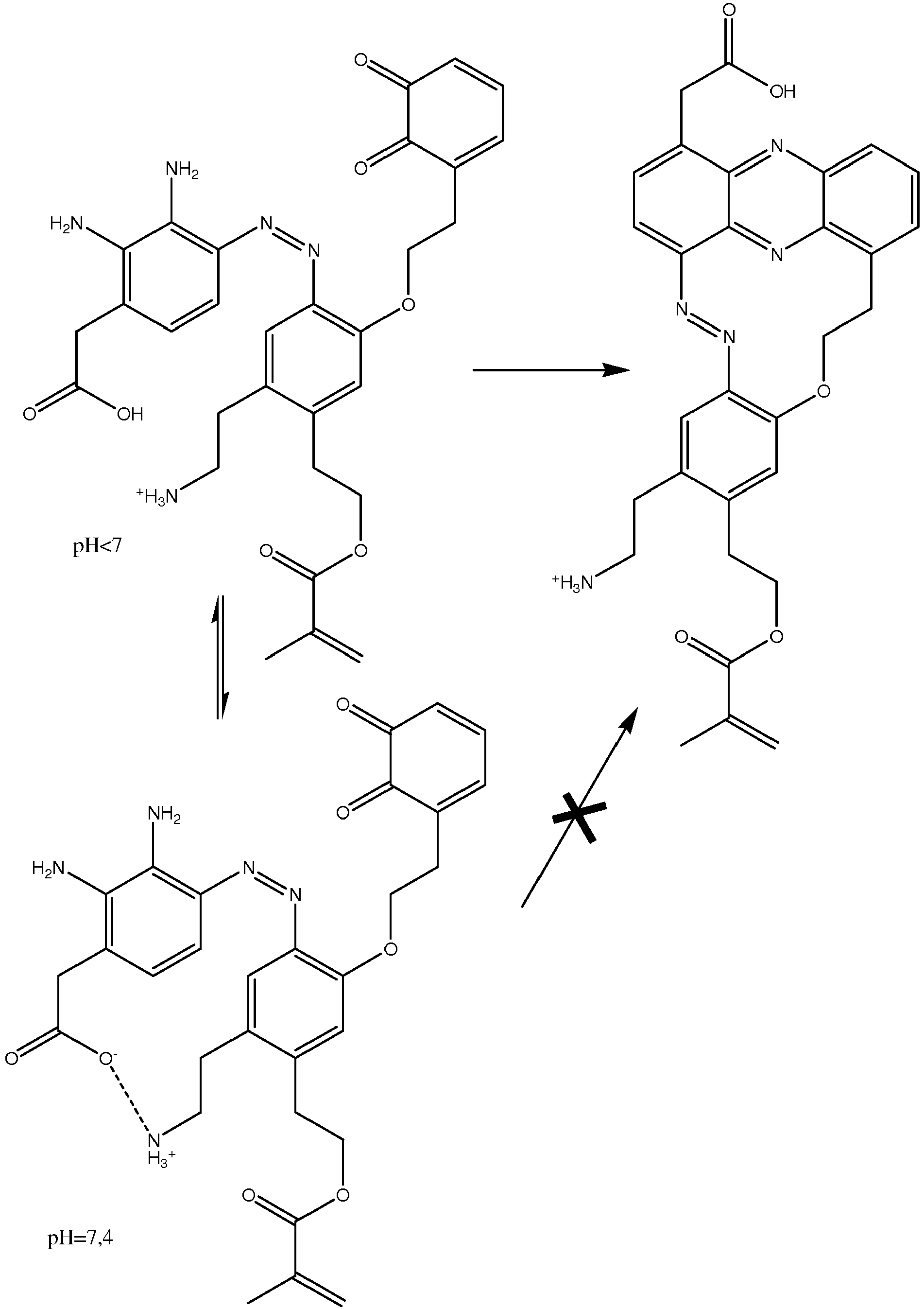

Im stabilisierten ersten Zustand ist eine intramolekulare Reaktion der zwei Keto- und Aminogruppen aus sterischen Gründen nicht möglich. Im zweiten Zustand können aber erneut die beiden ersten und zweiten Strukturelemente des ersten Paars unter Ringbildung kovalent unter Ringbildung zu einer Phenazinyl-Gruppe reagieren und damit den zweiten Zustand über einen alternativen Mechanismus irreversibel stabilisieren. Das bedeutet, dass der erste Zustand durch Einstellung eines etwa neutralen pH-Werts und Bildung eines vicinalen Zwitterions stabilisiert werden kann, da der pKa-Wert der Carboxy-Gruppe etwa 9,73 (Phenylessigsäure) beträgt, während der pKa-Wert der Aminogruppe etwa 4,31 (Phenylethylamin) beträgt. Dies bedeutet, dass der isoelektrische Punkt des Zwitterions ungefähr bei pH 7,4 liegt. Bei sauren pH-Werten <7 wird das Carboxylat-Ion protoniert und die Stabilisierung des ersten Zustands aufgehoben. Unter sauren Bedingungen ist der erste Zustand (cis-Konfiguration) instabiler und kann relativ einfach zum zweiten Zustand (trans-Konfiguration) umlagern bzw. umgelagert werden. Eine direkte Umwandlung aus dem stabilisierten ersten in den zweiten Zustand ist hingegen nicht möglich.

Im zweiten Zustand können dann die beiden ersten und zweiten Strukturelemente des ersten Paars miteinander reagieren und den zweiten Zustand durch intramolekulare Ringbildung, beispielsweise aufgrund einer photoinduzierten oder thermisch induzierten Reaktion, irreversibel stabilisieren. Alternativ kann die molekulare Schalterverbindung vor der Ringbildung durch erneutes Einstellen eines neutralen pH-Werts und gegebenenfalls durch weitere Reize wie UV-Bestrahlung wieder in den ersten Zustand zurückgeschaltet und in diesem durch intramolekulare Salzbildung wieder stabilisiert werden.

Alternativ oder zusätzlich zur Einstellung eines sauren pH-Werts kann der erste Zustand auch durch Einstellung einer hohen lonenstärke bzw. einer hohen lonenkonzentration destabilisiert werden. Beispielsweise kann das Zwitterion durch Zugabe von NaCl, das heißt durch Bereitstellung entsprechender Gegenionen, destabilisiert und in den zweiten Zustand umgelagert werden, da die intramolekulare Salzbildung zerstört wird:

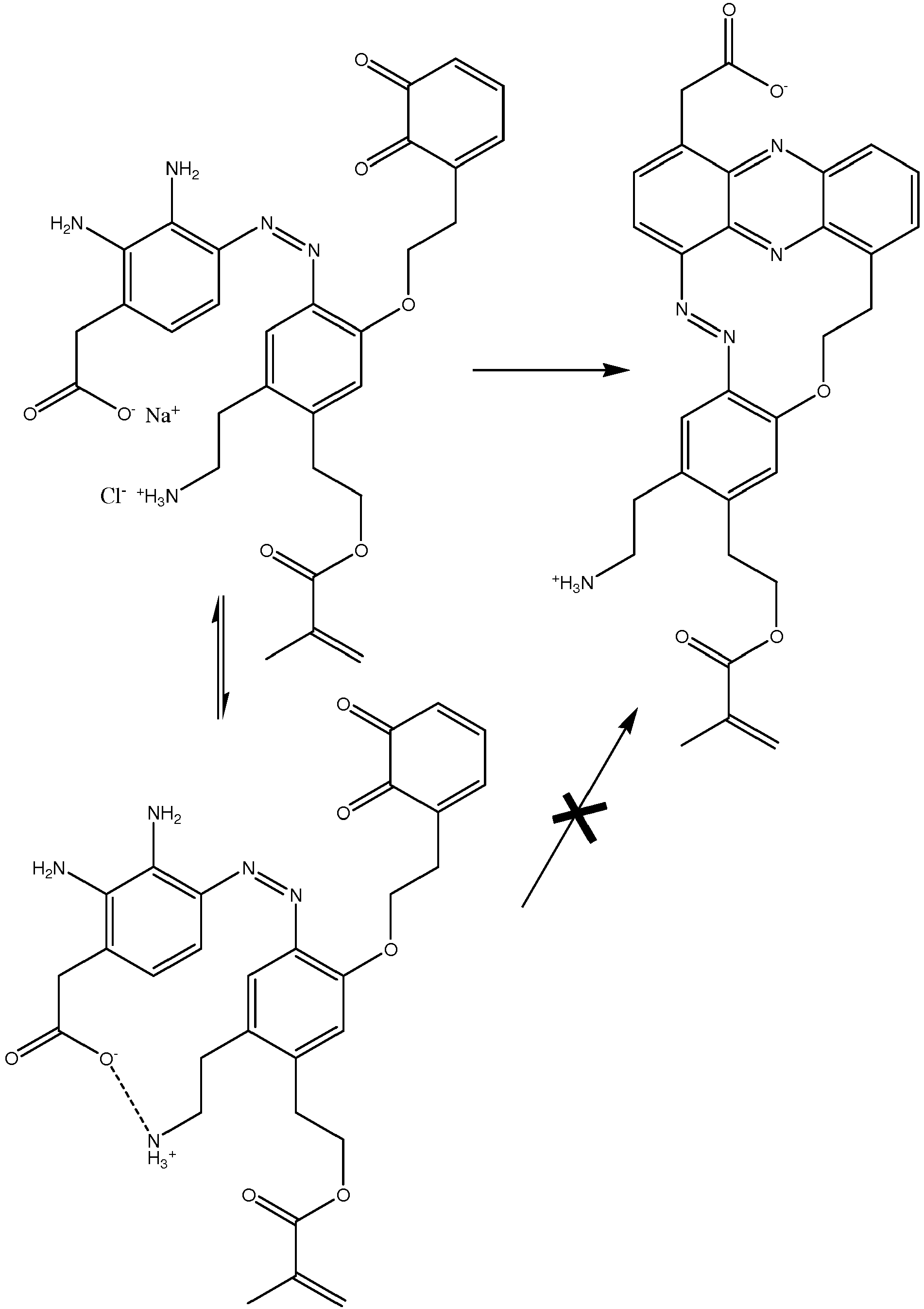

Schließlich umfasst die molekulare Schalterverbindung als viertes Strukturelement erneut die grundsätzlich optionale Methacrylat-Gruppe, über welche die Schalterverbindung kovalent mit weiteren Komponenten der ophthalmologischen Zusammensetzung, beispielsweise mit Acrylat- und/oder Methacrylat-Monomeren, vernetzt werden kann.

Unter physiologischen Bedingungen stabilisiert das Zwitterion somit den ersten Zustand, so dass eine photoinduzierte Umschaltung des molekularen Schalters in den zweiten Zustand verunmöglicht ist. Für die Modifizierung der Brechkraft des Linsenmaterials müssen daher die Umweltbedingungen so geändert werden, dass der stabilisierende Zwitterionen-Zustand aufgehoben wird. Hierzu kann im Fall wie beschrieben die Umgebung angesäuert und/oder die lonenstärke in der Umgebung erhöht werden. Dies kann im Rahmen einer Augen-Operation beim Einsetzen einer IOL durch Injektion einer sauren Lösung (beispielsweise HCl, Kohlensäure) oder durch die Injektion einer Salzlösung (z.B. NaCl) oder durch die Kombination von beidem in die vordere Kammer des Auges erfolgen.

Dabei sollte vermieden werden, dass das Augengewebe, insbesondere die Endothelzellen und die innere Schicht der Hornhaut geschädigt wird. Daher sollten der pH-Wert und die Ionenstärke so eingestellt bzw. gepuffert werden, dass es zu keiner Beschädigung des Augengewebes kommt. Während dieser künstlichen Bedingungen kann durch Bestrahlung die endgültige Brechkraft der IOL eingestellt werden. Schließlich stellen sich durch die natürliche Fluktuation des Kammerwassers wieder physiologische Bedingungen im Bereich der IOL ein, in denen nicht-umgesetzte molekulare Schalterverbindungen wieder über das sich erneut ausbildende Zwitterion im ersten Zustand stabilisiert werden.

Als Alternative zur Injektion von Salz und/oder Säure bzw. Base können auch Medikamente zur Manipulation des durch den Ziliarkörper produzierten Kammerwassers verwendet werden. Zum Beispiel vermittelt das Enzym Carboanhydrase, das in unpigmentierten und pigmentierten Ziliarepithel vorkommt, den Transport von Bicarbonat durch die reversible Hydratation von CO₂ zu HCO₃⁻ und Protonen (H⁺). Dieser Mechanismus kann ebenfalls zur vorübergehenden Ansäuerung des Kammerwassers verwendet werden, um ein vorzeitiges Umschalten des molekularen Schalters vom zweiten in den ersten Zustand zu verhindern.

Es ist grundsätzlich zu betonen, dass es zur Stabilisierung eines der Zustände ausreicht, wenn die Amino- und Carboxylat-Gruppen innerhalb des Moleküls im betreffenden Zustand nahe genug zueinander angeordnet sind, um eine ionische Brücke im Zwitterionenzustand zu bilden. Ferner können die ersten und zweiten Strukturelemente anhand ihrer pKa-Werte so gewählt werden, dass sie unter physiologischen Verbindungen ein Zwitterion und eine intramolekulare Ionenbindung bilden.

Ebenso ist die molekulare Schalterverbindung nicht auf die Verwendung von Carboxy- und Amino-Gruppen beschränkt. Beispielsweise können auch Sulfonsäuregruppen (-HSO₃ bzw. - SO₃⁻) als Säurekomponente vorgesehen sein.

### Ausführungsbeispiel 4

In einem weiteren Ausführungsbeispiel der Erfindung weist die molekulare Schalterverbindung im ersten Zustand die Formel 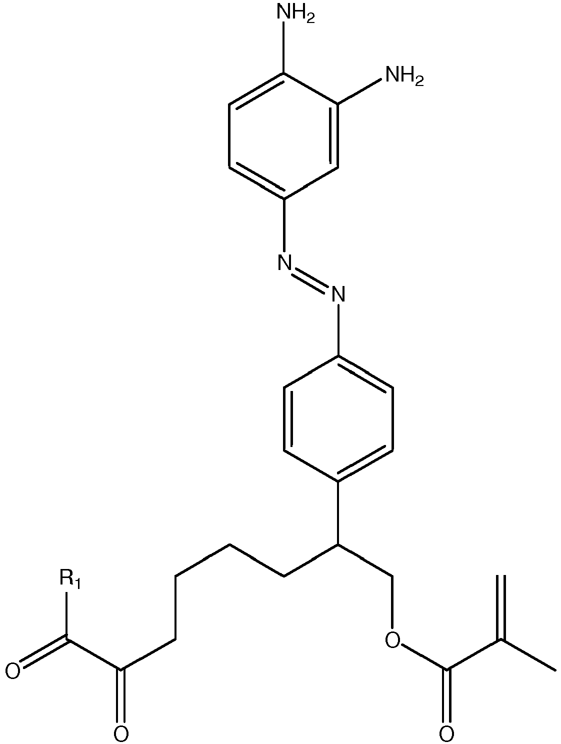 mit R₁=Wasserstoff oder CₙH₂ₙ₊₁ mit 1 ≤ n ≤ 30 und im zweiten Zustand die Formel 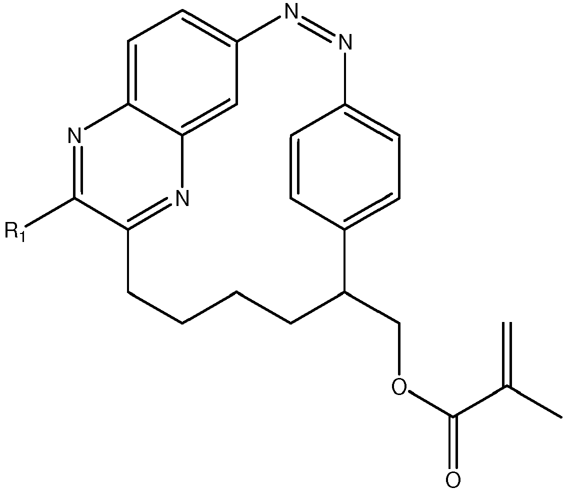 auf.

### Ausführunasbeispiel 5

In einem weiteren Ausführungsbeispiel der Erfindung ist vorgesehen, dass die molekulare Schalterverbindung im ersten Zustand die Formel 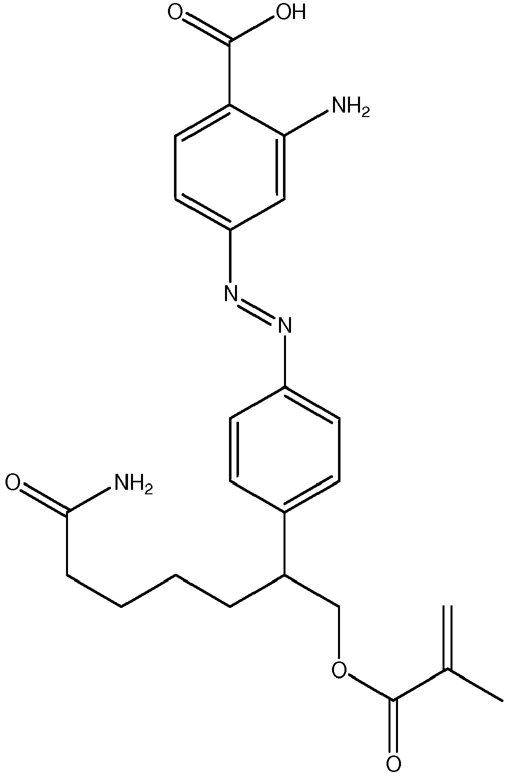 und/oder im zweiten Zustand die Formel 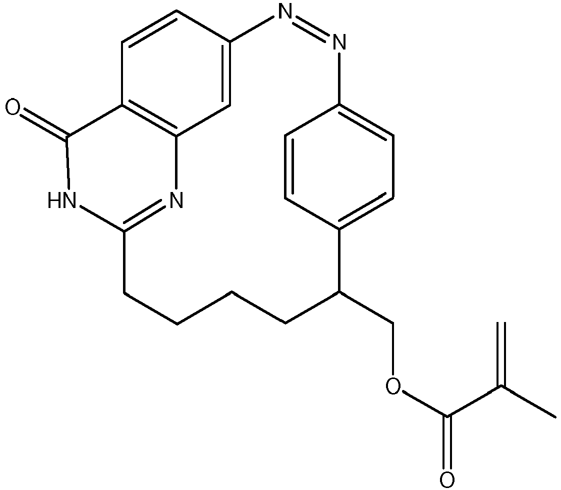 besitzt.

### Ausführunasbeispiel 6

In einem weiteren Ausführungsbeispiel der Erfindung ist vorgesehen, dass die molekulare Schalterverbindung im ersten Zustand die Formel 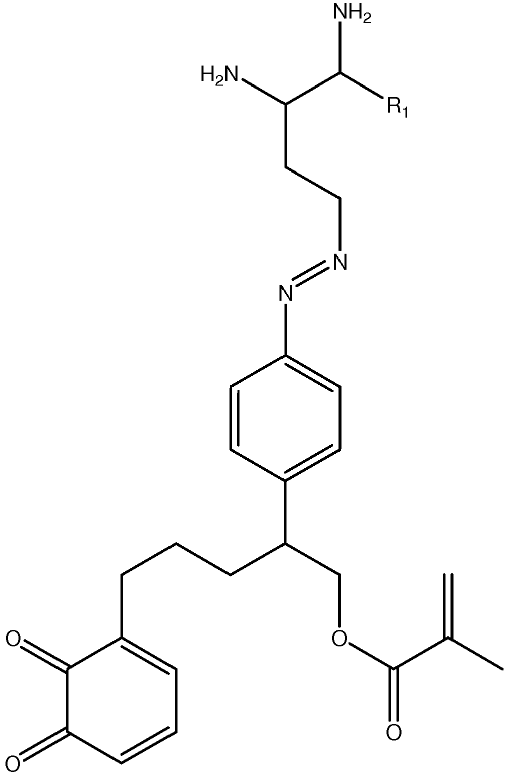 und/oder im zweiten Zustand die Formel 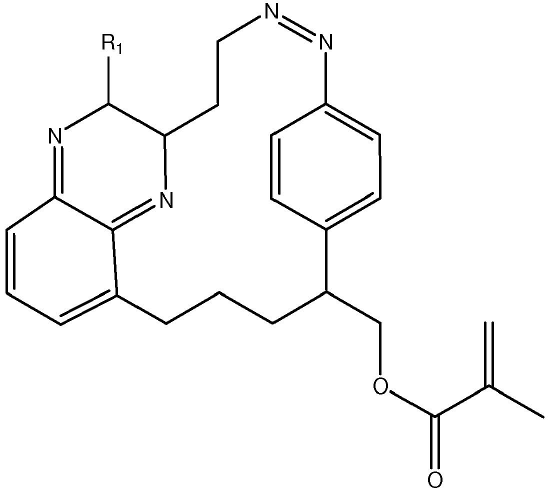 besitzt.

### Ausführungsbeispiel 7

In einem weiteren Ausführungsbeispiel der Erfindung ist vorgesehen, dass die molekulare Schalterverbindung im ersten Zustand die Formel 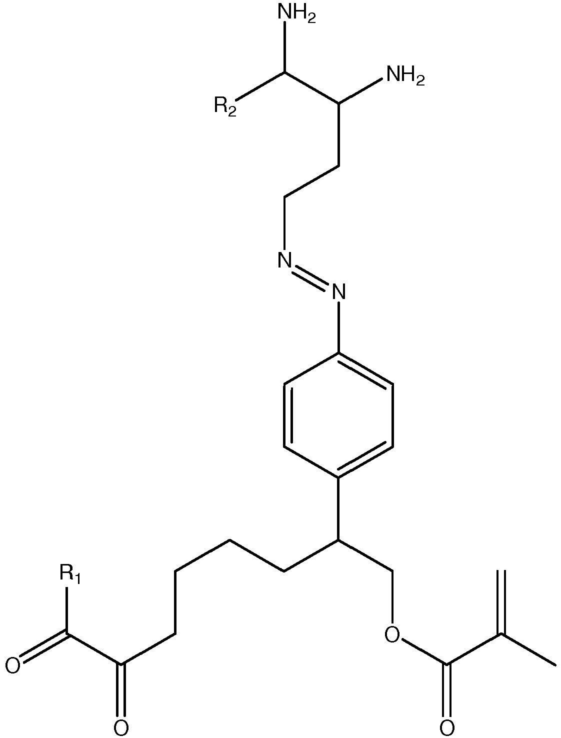 mit R₁ = H oder CₙH₂ₙ₊₁ mit 1 ≤ n ≤ 30 und im zweiten Zustand die Formel 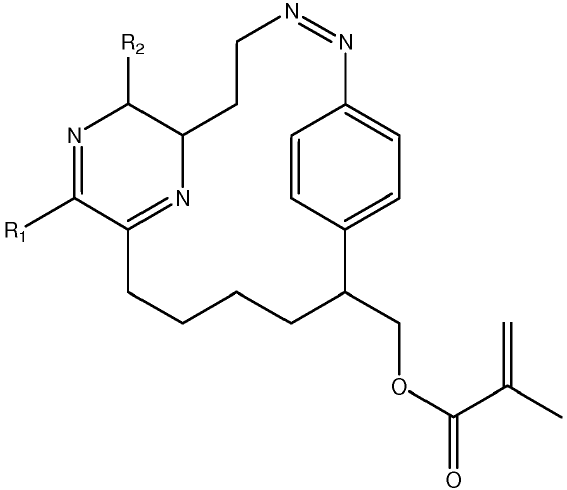 besitzt.

### Ausführunasbeispiel 8

Im Rahmen der vorliegenden Erfindung umfasst eine bevorzugte ophthalmologische Zusammensetzung für die optischen und/oder die haptischen Bereiche einer einteiligen oder mehrteiligen ophthalmologischen Linse wie etwa einer Intraokularlinse (IOL) ein Copolymer aus 2-Hydroxyethylmethacrylat (2-HEMA), 2-Ethoxyethylmethacrylat (EOEMA) und wenigstens einer molekularen Schalterverbindung. Dabei können beispielsweise eine oder mehrere der vorstehend genannten Schalterverbindungen verwendet werden.

Die Hydrogele eines solchen Copolymers haben einen Wassergehalt zwischen etwa 8 und etwa 40 Prozent bezogen auf das Gesamtgewicht der ophthalmologischen Zusammensetzung. Bevorzugt sind etwa 15 bis etwa 34 Massenprozente Wassergehalt bzw. Wasseraufnahmefähigkeit des Copolymers bezogen auf das Gesamtgewicht der ophthalmologischen Zusammensetzung, welche in einfachster Ausgestaltung nur aus den einzelnen Monomeren bzw. nur aus dem resultierenden Copolymer bestehen kann.

Eine als IOL ausgebildete ophthalmologische Linse der vorliegenden Erfindung kann durch grundsätzlich bekannte Verfahren aus der ophthalmologischen Zusammensetzung hergestellt werden. Beispielsweise wird zunächst eine flüssige Mischung aus den gewünschten Monomeren und einem Initiator in eine röhrenförmige bzw. zylindrische Form eingefüllt. Neben einem Initiator bzw. Vernetzer können zusätzlich auch UV-Absorber und/oder Violett-Absorber (Gelbfarbstoffe) der Mischung beigefügt oder in die resultierende Polymermatrix eingearbeitet werden. Aus dem auspolymerisierten Zylinder oder Stab werden dann in an sich bekannter Weise Scheiben bzw. IOL-Rohlinge geschnitten und anschließend geschliffen oder geläppt. Ebenso kann eine Wärmebehandlung des auspolymerisierten Zylinders, der IOL-Rohlinge und/oder der fertigen IOL vorgesehen sein. Die IOLs können grundsätzlich einteilig oder mehrteilig ausgebildet sein, das heißt neben einem optischen auch mindestens einen haptischen Teil aufweisen, wobei der optische und der haptische Teil grundsätzlich aus demselben Material oder aus unterschiedlichen Materialien bestehen können.

Eine erfindungsgemäße ophthalmologische Zusammensetzung kann beispielsweise folgende Komponenten umfassen:

| | |
|---|---|
| HEMA (2-Hydroxyethylmethacrylat): | 60-90 % |
| Molekulare Schalterverbindung: | 0,1-30 % |
| EOEMA (2-Ethoxyethylmethacrylat): | 0-30 % |
| EGDMA (Ethylenglykoldimethacrylat): | 0,25-1,5 % |
| Radikalstarter | 0,01-0,1 % |
| UV-Absorber: | 0-1,0 % |
| Violett-Absorber: | 0-0,16 % |

Im Rahmen der vorliegenden Erfindung sind Prozentangaben grundsätzlich als Massenprozente zu verstehen, sofern nichts anderes angegeben ist. Es versteht sich, dass die Massenprozente aller Komponenten stets so zu wählen sind, dass sie sich immer und ausschließlich zu 100 % ergänzen.

Zur Herstellung einer IOL wurden 750 g HEMA mit 195 g EOEMA, 35 g molekularer Schalterverbindungen, 0,5 g-0,7 g Radikalstarter (z. B. 2,2-Azobis(2,4-dimethylvaleronitril) (Du-Pont Vazo 52) oder α,α'-Azoisobutyronitril (AIBN)) und etwa 0,1 g-0,3 g Ethylenglycoldimethacrylat (EGDMA) gemischt. Als molekulare Schalterverbindung können beispielsweise die in den vorhergehenden Ausführungsbeispielen beschriebenen Schalterverbindungen einzeln oder in beliebiger Kombination verwendet werden. Das Gemisch wurde unter kräftigem Rühren entgast, in zylindrische Formen gegossen, bei 30 °C für etwa 12 Stunden polymerisiert und bei etwa 100 °C für 6 Stunden nachgehärtet. Aus dem auspolymerisierten Zylinder wurden dann in an sich bekannter Weise IOL-Rohlinge geformt und bei etwa 100 °C erneut für 5 bis 6 Stunden gehärtet. Nach dem Härten wurden die IOL-Rohlinge durch Schleifen bzw. Läppen zu den fertigen IOLs weiterverarbeitet. Die derart hergestellten IOLs besitzen eine Wasseraufnahmefähigkeit zwischen etwa 26 und etwa 30 %.

Durch Variierung des Anteils an molekularer Schalterverbindung am Gesamtgewicht der ophthalmologischen Zusammensetzung kann der Brechungsindex der späteren IOL in weiten Bereichen nachträglich eingestellt werden. Beispielsweise kann ein Teil des EOEMA durch die molekulare Schalterverbindung ersetzt werden, um den Brechungsindex der IOL um bis zu 0,14 zu erhöhen. Das vorstehend beschriebene Verhältnis von etwa 85 % EOEMA zu etwa 15 % molekularer Schalterverbindung erlaubt eine Änderung der Refraktion einer IOL von 14,5 D auf 15 D, wenn etwa 30 % der molekularen Schalterverbindung beispielsweise durch Lichtbestrahlung zur Umlagerung und intramolekularen Reaktion gebracht wird. Schaltet man etwa 50 % der molekularen Schalterverbindung zwischen dem ersten und dem zweiten Zustand, ist eine Änderung auf 15,3 D möglich, während ein Schalten von 100 % der molekularen Schalterverbindung eine Änderung auf 16 D erlaubt. Wenn man im oben genannten Beispiel EOEMA vollständig durch die molekulare Schalterverbindung ersetzt, kann beim Schalten von 100 % der Schalterverbindung eine maximale Änderung von ca. 10 D erzielt werden. Reagieren nur 50 % der Schalterverbindung ist entsprechend eine Änderung von etwa 5,4 D möglich. Wählt man im oben genannte Beispiel eine Mischung von 115 g EOEMA und 115 g molekularer Schalterverbindung (Verhältnis 1:1), können beim nachträglichen Schalten der molekularen Schalterverbindung Änderungen von etwa 2,7 D erzielt werden. Bei der postoperativen Einstellung einer IOL sind insbesondere nachträglich einstellbare Differenzen von 0,5 D bis 1 D therapeutisch sinnvoll, so dass in der Regel ein Anteil von etwa 10 % bis 20 % an molekularer Schalterverbindung bezogen auf die Gesamtmasse der ophthalmologischen Zusammensetzung ausreicht.

### Ausführungsbeispiel 9

Eine erfindungsgemäße ophthalmologische Zusammensetzung gemäß einem weiteren Ausführungsbeispiel enthält folgende Komponenten:

| | |
|---|---|
| EA (Ethylacrylat): | 45-75 % |
| EMA (Ethylmethacrylat): | 25-50 % |
| TFEMA (Trifluorethylmethacrylat): | 1-10 % |
| EGDMA (Ethylenglykoldimethacrylat): | 0,25-1,5 % |
| Radikalstarter: | 0,01-0,1 % |
| UV-Absorber: | 0-1,0 % |
| Violett-Absorber: | 0-0,16 % |
| Molekulare Schalterverbindung: | 0,1-10 % |

Der Glaspunkt dieses hydrophoben Polymers liegt bevorzugt im Bereich 0-11 °C. Als molekulare Schalterverbindung können die in den vorhergehenden Ausführungsbeispielen beschriebenen Schalterverbindungen einzeln oder in beliebiger Kombination verwendet werden.

### Ausführungsbeispiel 10

Es wurde eine ophthalmologische Linse aus einer ophthalmologischen Zusammensetzung hergestellt, wobei die ophthalmologische Zusammensetzung folgende Komponenten umfasst:

| | |
|---|---|
| HEMA (2-Hydroxyethylmethacrylat): | 50-90 % |
| MMA (Methylmethacrylat): | 0-16 % |
| EGDMA (Ethylenglykoldimethacrylat): | 0,25-1,5 % |
| Radikalstarter: | 0,01-0,1 % |
| UV-Absorber: | 0,1-1,0 % |
| Violett-Absorber: | 0-0,16 % |
| Molekulare Schalterverbindung: | 0,1-30 % |

Der Wassergehalt des hydratisierten Polymers liegt bevorzugt bei 26-30 %. Als molekulare Schalterverbindung können die in den vorhergehenden Ausführungsbeispielen beschriebenen Schalterverbindungen einzeln oder in beliebiger Kombination verwendet werden.

Es ist zu betonen, dass die ophthalmologische Zusammensetzung nicht auf die Verwendung bestimmter Monomere beschränkt ist. Alternativ oder zusätzlich zu (Meth)acrylaten können auch andere Monomere wie etwa Silikone vorgesehen sein.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

## Patentansprüche

1. Ophthalmologische Zusammensetzung mit wenigstens einer molekularen Schalterverbindung, welche mittels eines Reizes zwischen einem ersten und einem zweiten Zustand schaltbar ist, wobei die molekulare Schalterverbindung im ersten und im zweiten Zustand unterschiedliche Brechungsindizes besitzt,
**dadurch gekennzeichnet, dass**
die molekulare Schalterverbindung zumindest ein erstes Strukturelement und ein korrespondierendes zweites Strukturelement umfasst, welche nur in einem der Zustände intramolekular miteinander reagieren können.

2. Ophthalmologische Zusammensetzung nach Anspruch 1,
**dadurch gekennzeichnet, dass**
wenigstens zwei Paare aus erstem und zweitem Strukturelement vorgesehen sind, wobei eines der Paare nur im ersten Zustand intramolekular miteinander reagieren kann und das andere Paar nur im zweiten Zustand intramolekular miteinander reagieren kann.

3. Ophthalmologische Zusammensetzung nach Anspruch 1 oder 2,
**dadurch gekennzeichnet, dass**
die molekularen Schalterverbindung wenigstens ein drittes Strukturelement umfasst, das beim Schalten zwischen dem ersten und dem zweiten Zustand seine Konfiguration vorzugsweise reversibel ändert.

4. Ophthalmologische Zusammensetzung nach einem der Ansprüche 1 bis 3,
**dadurch gekennzeichnet, dass**
die molekulare Schalterverbindung durch Temperaturänderung und/oder durch Bestrahlung mit elektromagnetischer Strahlung zwischen dem ersten und dem zweiten Zustand schaltbar ist.

5. Ophthalmologische Zusammensetzung nach einem der Ansprüche 1 bis 4,
**dadurch gekennzeichnet, dass**
das erste Strukturelement und das zweite Strukturelement in Abhängigkeit eines pH-Werts und/oder einer lonenkonzentration der ophthalmologischen Zusammensetzung intramolekular reagieren können.

6. Ophthalmologische Zusammensetzung nach einem der Ansprüche 1 bis 5,
**dadurch gekennzeichnet, dass**
die molekularen Schalterverbindung wenigstens ein viertes Strukturelement umfasst, mittels welchem die molekulare Schalterverbindung polymerisierbar ist.

7. Ophthalmologische Zusammensetzung nach einem der Ansprüche 1 bis 6,
**dadurch gekennzeichnet, dass**
die molekulare Schalterverbindung im ersten Zustand die allgemeine Formel I besitzt
R₁-Z1-R2 (I),
in welcher
Z₁ (E) -N=N-, (Z) -N=N-, (E) -C=C- oder (Z) -C=C- bedeutet,
R₁ ausgewählt ist aus der Gruppe 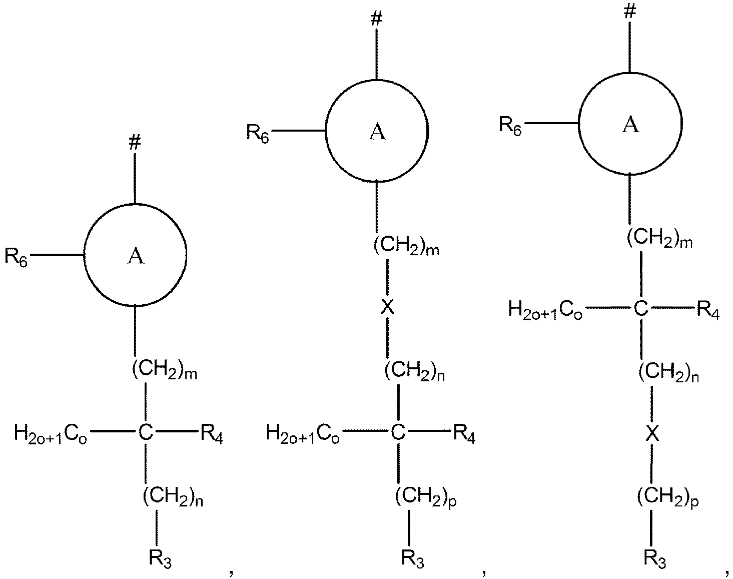 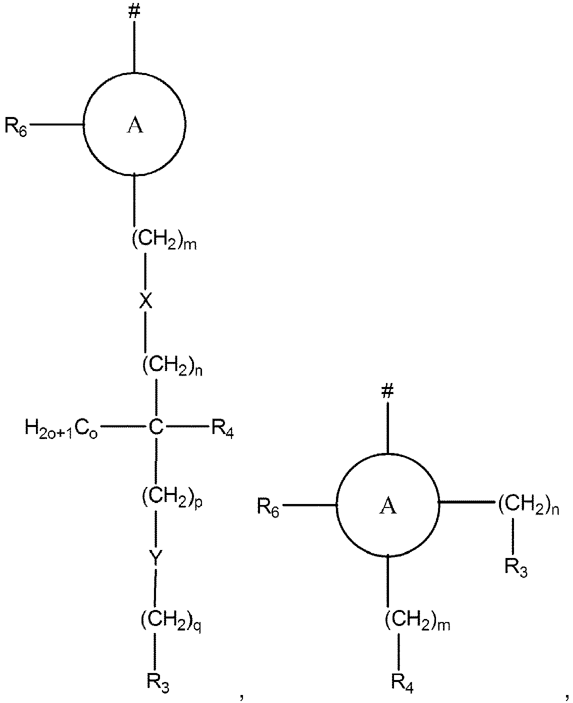 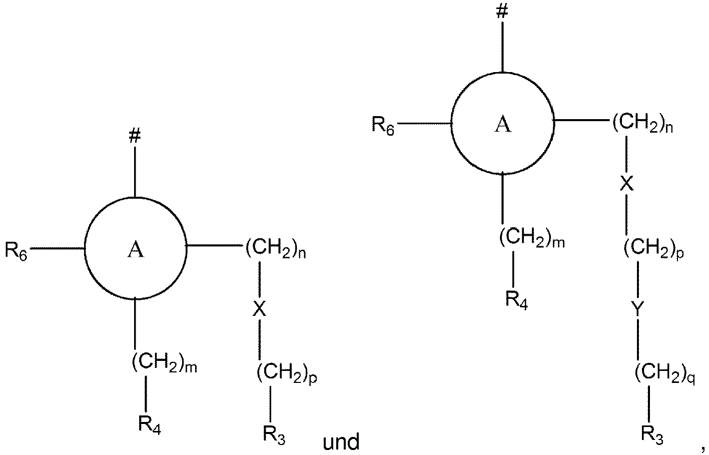 wobei
# die Verknüpfungsstelle mit Z1 bezeichnet,
m, n, o, p und q in R₁ jeweils unabhängig voneinander eine Ganzzahl zwischen 0 und 30 bedeuten,
X und Y in R₁ unabhängig voneinander ausgewählt sind aus CH₂, O, S oder NR₅, wobei R₅ CᵣH₂ᵣ₊₁ mit 0≤r≤30 bedeutet,
A ausgewählt ist aus der Gruppe 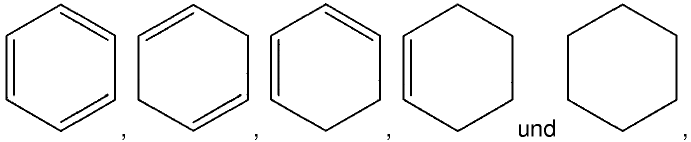
R₂ und R₃ abhängig voneinander ausgewählt sind aus der Gruppe 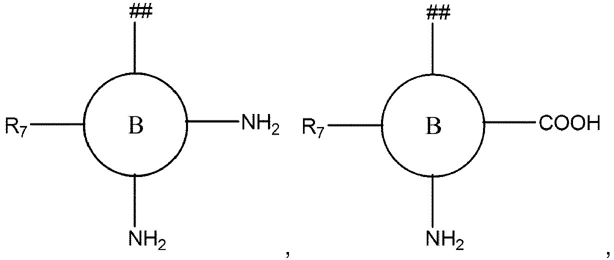 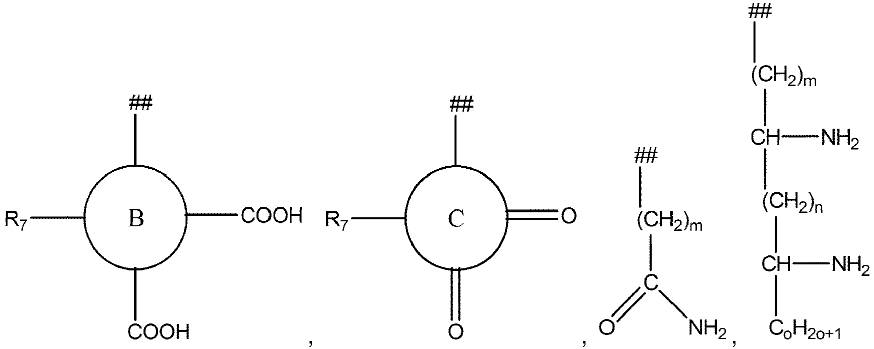 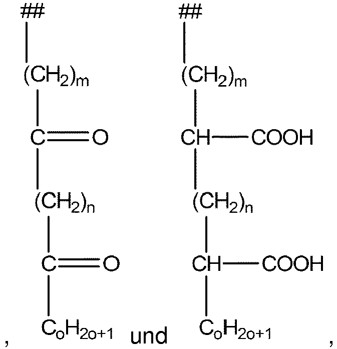 so dass R₂ und R₃ in einem der beiden Zustände intramolekular miteinander reagieren können, wobei
## im Fall von R₂ die Verknüpfungsstelle mit Z₁ und im Fall von R₃ die Verknüpfungsstelle mit R₁ bezeichnet,
m, n und o in R₂ und R₃ jeweils unabhängig voneinander eine Ganzzahl zwischen 0 und 30 bedeuten,
B ausgewählt ist aus der Gruppe 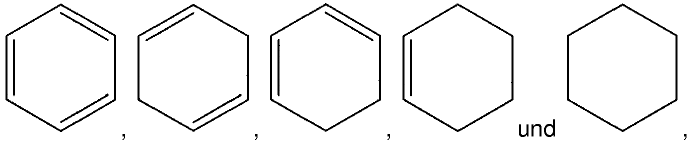
C ausgewählt ist aus der Gruppe 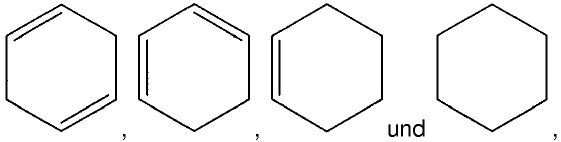
R₄ ausgewählt ist aus der Gruppe 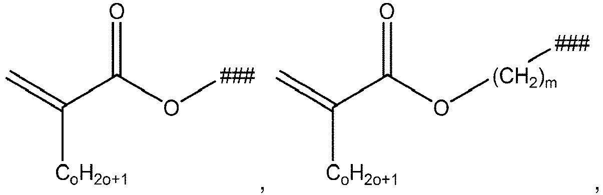 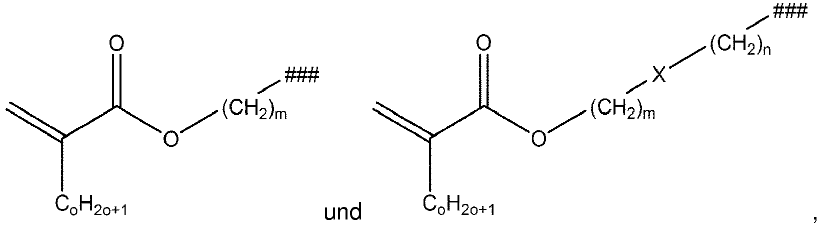 wobei
### die Verknüpfungsstelle mit R₁ bezeichnet,
X in R₄ ausgewählt ist aus CH₂, O, S oder NR₅, wobei R₅ CᵣH₂ᵣ₊₁ mit 0≤r≤30 bedeutet,
m, n und o in R₄ jeweils unabhängig voneinander eine Ganzzahl zwischen 0 und 30 bedeuten, und
R₆ und R₇ ausgewählt sind aus 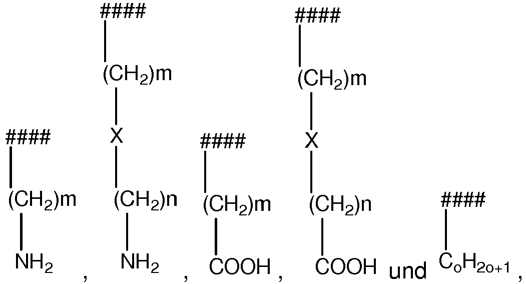 wobei
#### im Fall von R₆ die Verknüpfungsstelle mit A und im Fall von R₇ die Verknüpfungsstelle mit B oder C bezeichnet,
m, n und o in R₆ und R₇ jeweils unabhängig voneinander eine Ganzzahl zwischen 0 und 30 bedeuten und
X in R₆ und R₇ unabhängig voneinander ausgewählt sind aus CH₂, O, S oder NR₅, wobei R₅ CᵣH₂ᵣ₊₁ mit 0≤r≤30 bedeutet.

8. Ophthalmologische Linse, umfassend eine ophthalmologische Zusammensetzung nach einem der Ansprüche 1 bis 7.

9. Ophthalmologische Linse nach Anspruch 8,
**dadurch gekennzeichnet, dass**
diese als Intraokularlinse oder als Kontaktlinse oder als Brillenglas ausgebildet ist.

10. Verfahren zum Verändern einer Brechkraft einer ophthalmologischen Linse, welche eine ophthalmologische Zusammensetzung nach einem der Ansprüche 1 bis 7 umfasst und/oder gemäß einem der Ansprüche 8 oder 9 ausgebildet ist, bei welchem die wenigstens eine molekularen Schalterverbindung mittels eines Reizes zwischen einem ersten und einem zweiten Zustand geschaltet wird, wodurch sich die Brechkraft der ophthalmologischen Linse ändert, wobei das zumindest eine erste Strukturelement und das korrespondierende zweite Strukturelement nur in einem der Zustände intramolekular miteinander zur Reaktion gebracht werden.

## Claims

1. Ophthalmological composition comprising at least one molecular switch compound which can be switched by means of a stimulus between a first and a second state, the molecular switch compound possessing different refractive indices in the first and in the second state,
**characterized in that**
the molecular switch compound comprises at least a first structural element and a corresponding second structural element, which are able to react with one another intramolecularly only in one of the states.

2. Ophthalmological composition according to Claim 1,
**characterized in that**
at least two pairs of first and second structural element are provided, with one of the pairs being able to react with one another intramolecularly only in the first state and the other pair being able to react with one another intramolecularly only in the second state.

3. Ophthalmological composition according to Claim 1 or 2,
**characterized in that**
the molecular switch compound comprises at least one third structural element, which changes its configuration, preferably reversibly, on switching between the first and the second state.

4. Ophthalmological composition according to any of Claims 1 to 3,
**characterized in that**
the molecular switch compound can be switched between the first and the second state by temperature change and/or by irradiation with electromagnetic radiation.

5. Ophthalmological composition according to any of Claims 1 to 4,
**characterized in that**
the first structural element and the second structural element are able to react intramolecularly in dependence on a pH and/or an ion concentration of the ophthalmological composition.

6. Ophthalmological composition according to any of Claims 1 to 5,
**characterized in that**
the molecular switch compound comprises at least one fourth structural element, by means of which the molecular switch compound is polymerizable.

7. Ophthalmological composition according to any of Claims 1 to 6,
**characterized in that**
the molecular switch compound in the first state possess the general formula I
R₁-Z1-R₂ (I),
in which
Z1 is (E)-N=N-, (Z) is -N=N-, (E) is -C=C- or (Z) is -C=C-,
R₁ is selected from the group 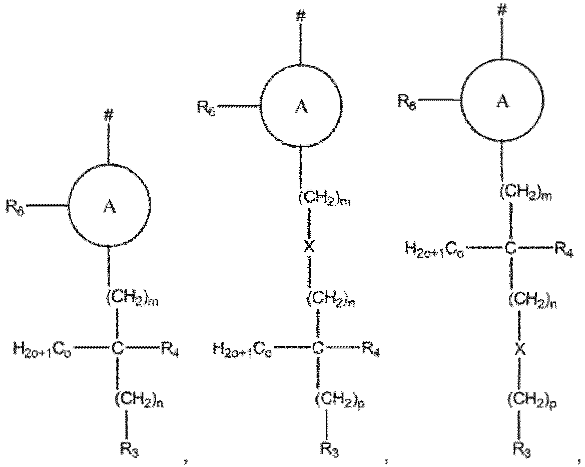 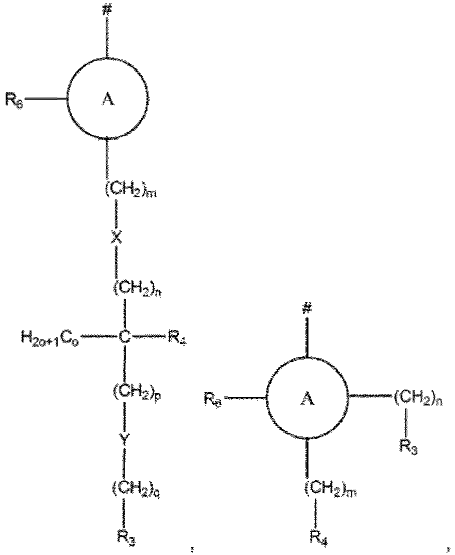 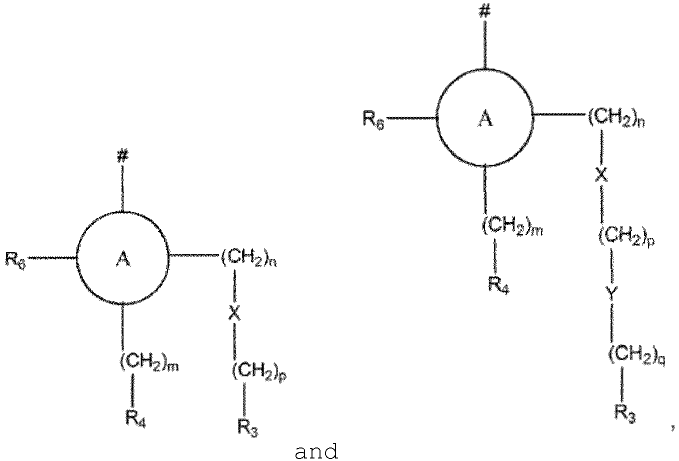 where
# denotes the linkage site to Z1,
m, n, o, p and q in R₁ in each case independently of one another are an integer between 0 and 30,
X and Y in R₁ independently of one another are selected from CH₂, O, S or NR₅, where R₅ is CᵣH₂ᵣ₊₁ with 0≤r≤30,
A is selected from the group 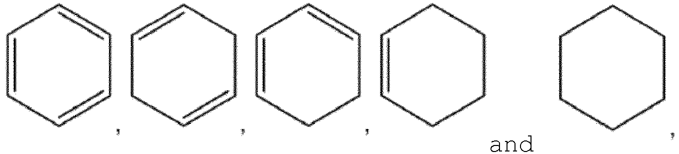
R₂ and R₃ dependently of one another are selected from the group 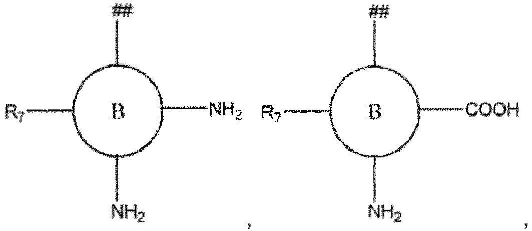 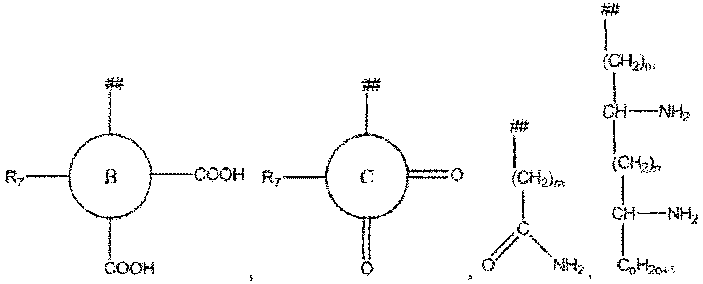 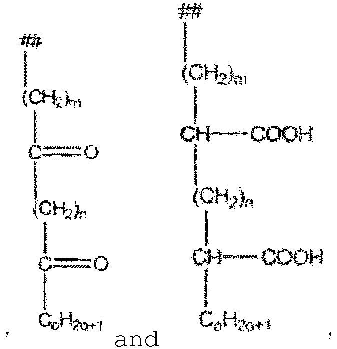 so that R₂ and R₃ are able to react with one another intramolecularly in one of the two states, where
## in the case of R₂ denotes the linkage site to Z1 and in the case of R₃ denotes the linkage site to R₁,
m, n and o in R₂ and R₃ in each case independently of one another are an integer between 0 and 30,
B is selected from the group 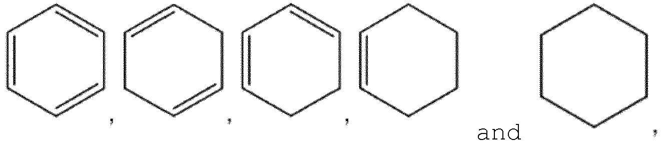
C is selected from the group 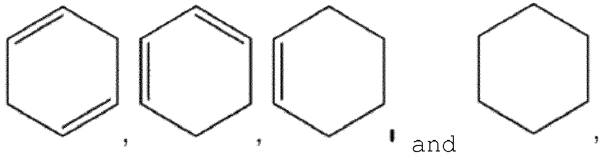
R₄ is selected from the group 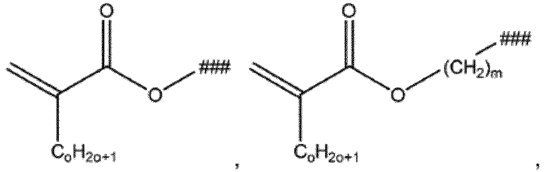 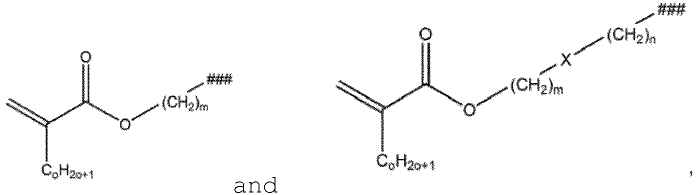
where
### denotes the linkage site to R₁,
X in R₄ is selected from CH₂, O, S or NR₅, where R₅ is CᵣH₂ᵣ₊₁ with 0≤r≤30,
m, n and o in R₄ in each case independently of one another are an integer between 0 and 30, and
R₆ and R₇ are selected from 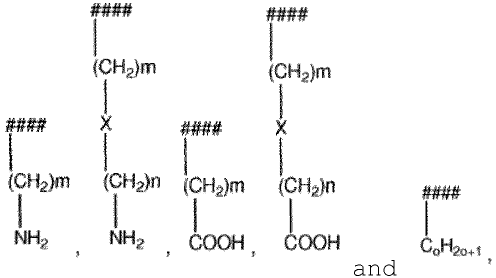
where
#### in the case of R₆ denotes the linkage site to A and in the case of R₇ denotes the linkage site to B or C,
m, n and o in R₆ and R₇ in each case independently of one another are an integer between 0 and 30, and
X in R₆ and R₇ independently of one another are selected from CH₂, O, S or NR₅, where R₅ is CᵣH₂ᵣ₊₁ with 0≤r≤30.

8. Ophthalmological lens comprising an ophthalmological composition according to any of Claims 1 to 7.

9. Ophthalmological lens according to Claim 8,
**characterized in that**
it is configured as an intraocular lens or as a contact lens or as a spectacle lens.

10. Method for altering a refractive power of an ophthalmological lens, which comprises an ophthalmological composition according to any of Claims 1 to 7 and/or is configured according to either of Claims 8 and 9, wherein the at least one molecular switch compound is switched by means of a stimulus between a first and a second state, causing the refractive power of the ophthalmological lens to change, where the at least one first structural element and the corresponding second structural element are brought to reaction with one another intramolecularly only in one of the states.

## Revendications

1. Composition ophtalmologique contenant au moins un composé commutateur moléculaire, qui peut être commuté entre un premier et un deuxième état au moyen d'un stimulus, le composé commutateur moléculaire présentant un indice de réfraction différent au premier et au deuxième état,
**caractérisée en ce que**
le composé commutateur moléculaire comprend au moins un premier élément structural et un deuxième élément structural correspondant, qui ne peuvent réagir intramoléculairement l'un avec l'autre qu'à un des états.

2. Composition ophtalmologique selon la revendication 1, **caractérisée en ce qu'**au moins deux paires de premier et deuxième élément structural sont prévues, une des paires ne pouvant réagir intramoléculairement qu'au premier état et l'autre paire ne pouvant réagir intramoléculairement qu'au deuxième état.

3. Composition ophtalmologique selon la revendication 1 ou 2, **caractérisée en ce que** le composé commutateur moléculaire comprend au moins un troisième élément structural, qui modifie de préférence de manière réversible sa configuration lors de la commutation entre le premier et le deuxième état.

4. Composition ophtalmologique selon l'une quelconque des revendications 1 à 3, **caractérisée en ce que** le composé commutateur moléculaire peut être commuté entre le premier et le deuxième état par modification de la température et/ou par exposition à un rayonnement électromagnétique.

5. Composition ophtalmologique selon l'une quelconque des revendications 1 à 4, **caractérisée en ce que** le premier élément structural et le deuxième élément structural peuvent réagir intramoléculairement en fonction d'une valeur de pH et/ou d'une concentration ionique de la composition ophtalmologique.

6. Composition ophtalmologique selon l'une quelconque des revendications 1 à 5, **caractérisée en ce que** le composé commutateur moléculaire comprend au moins un quatrième élément structural, au moyen duquel le composé commutateur moléculaire peut être polymérisé.

7. Composition ophtalmologique selon l'une quelconque des revendications 1 à 6, **caractérisée en ce que** le composé commutateur moléculaire présente au premier état la formule générale I
R₁-Z1-R2 (I),
dans laquelle
Z₁ signifie (E) -N=N-, (Z) -N=N-, (E) -C=C- ou (Z) -C=C-,
R₁ est choisi dans le groupe constitué par 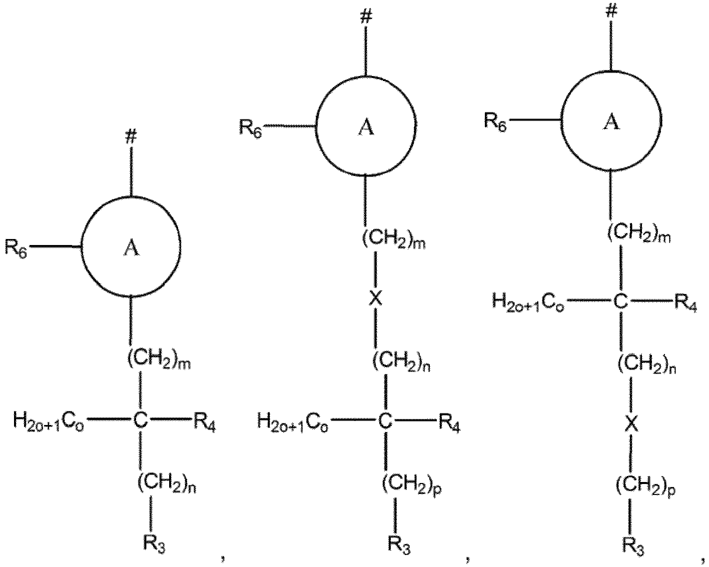 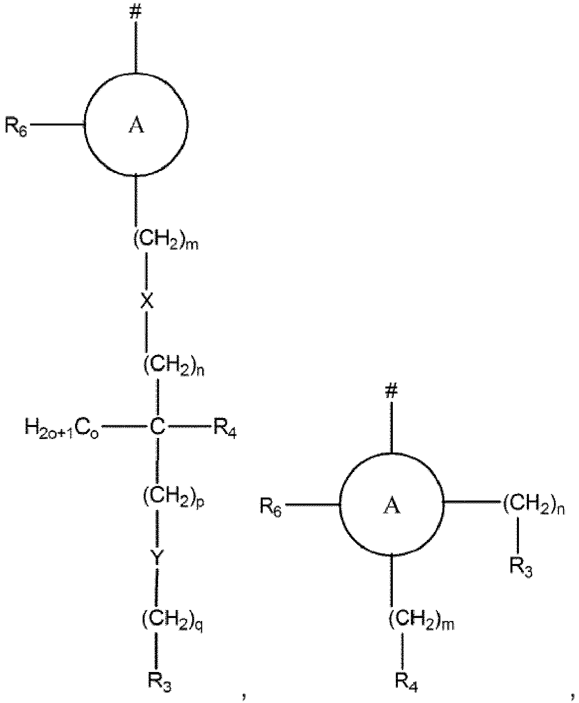 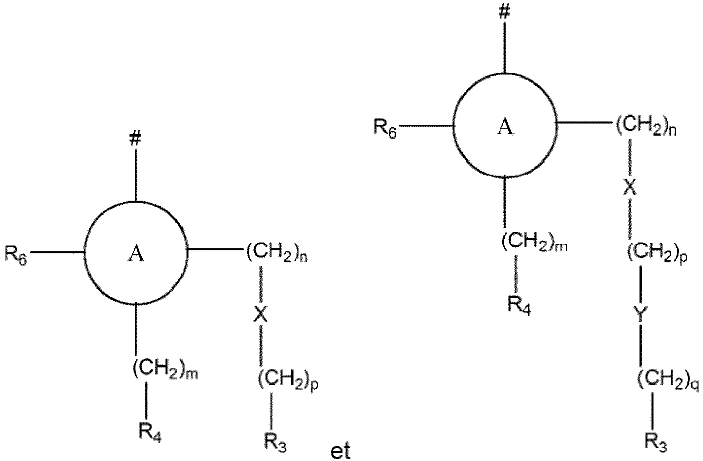
# désignant l'emplacement de liaison avec Z1,
m, n, o, p et q dans R₁ signifiant chacun indépendamment les uns des autres un nombre entier compris entre 0 et 30,
X et Y dans R₁ étant chacun choisis indépendamment l'un de l'autre parmi CH₂, O, S ou NR₅, R₅ signifiant CᵣH₂ᵣ₊₁ avec 0 ≤ r ≤ 30,
A étant choisi dans le groupe constitué par : 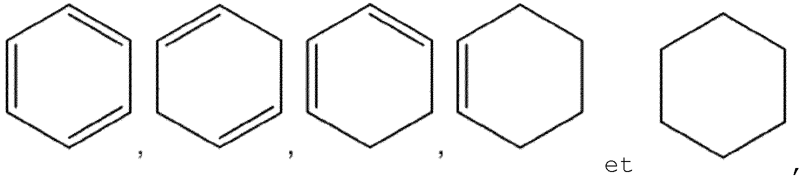
R₂ et R₃ étant choisis dépendamment l'un de l'autre dans le groupe constitué par : 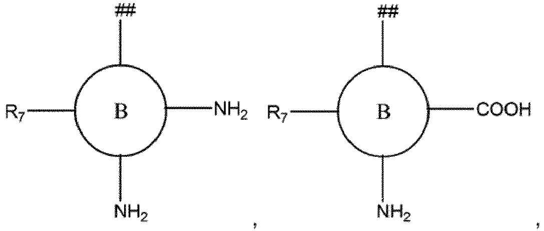 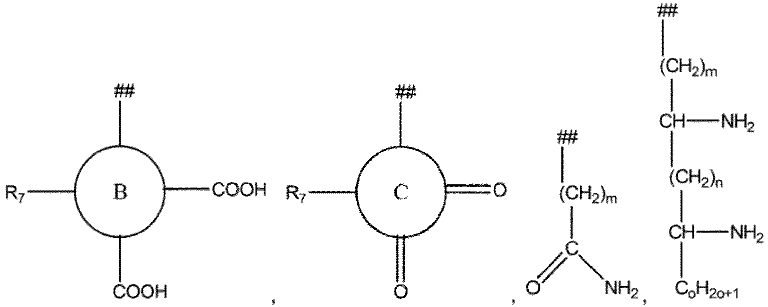 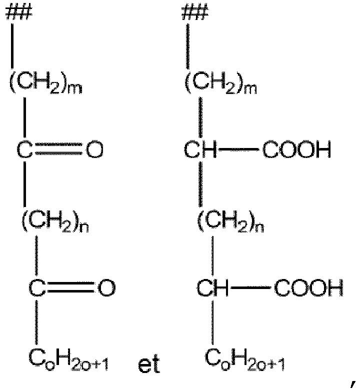 de telle sorte que R₂ et R₃ puissent réagir intramoléculairement l'un avec l'autre à un des deux états,
## dans le cas de R₂ désignant l'emplacement de liaison avec Z₁ et, dans le cas de R₃, l'emplacement de liaison avec R₁,
m, n et o dans R₂ et R₃ signifiant chacun indépendamment les uns des autres un nombre entier compris entre 0 et 30,
B étant choisi dans le groupe constitué par : 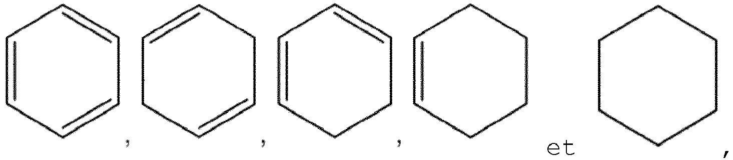
C étant choisi dans le groupe constitué par : 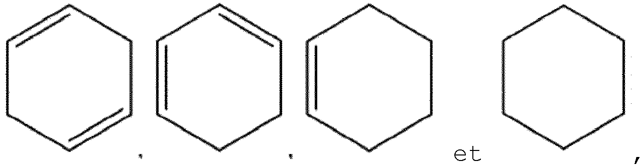
R₄ étant choisi dans le groupe constitué par : 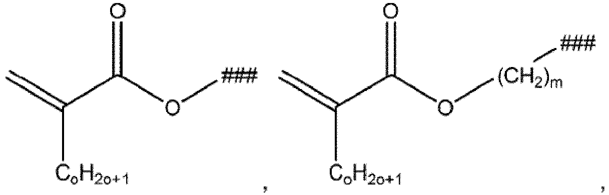 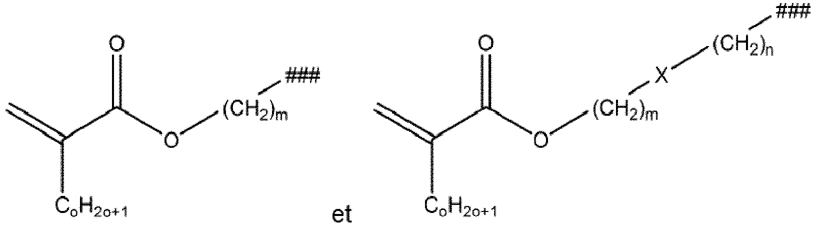
### désignant l'emplacement de liaison avec R₁,
X dans R₄ étant choisi parmi CH₂, O, S ou NR₅, R₅ signifiant CᵣH₂ᵣ₊₁ avec 0 ≤ r ≤ 30,
m, n et o dans R₄ signifiant chacun indépendamment les uns des autres un nombre entier compris entre 0 et 30, et
R₆ et R₇ étant choisis parmi : 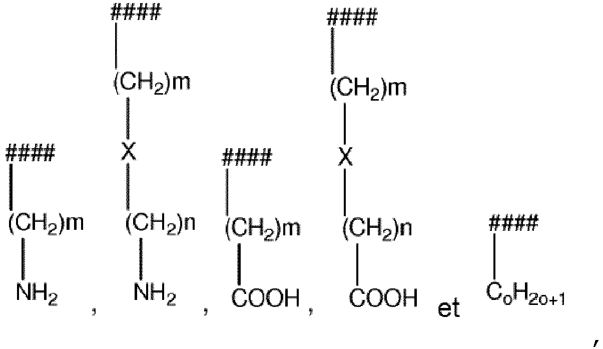
#### dans le cas de R₆ désignant l'emplacement de liaison avec A et, dans le cas de R₇, l'emplacement de liaison avec B ou C,
m, n et o dans R₆ et R₇ signifiant chacun indépendamment les uns des autres un nombre entier compris entre 0 et 30, et
les X dans R₆ et R₇ étant choisis indépendamment les uns des autres parmi CH₂, O, S ou NR₅, R₅ signifiant CᵣH₂ᵣ₊₁ avec 0 ≤ r ≤ 30.

8. Lentille ophtalmologique, comprenant une composition ophtalmologique selon l'une quelconque des revendications 1 à 7.

9. Lentille ophtalmologique selon la revendication 8, **caractérisée en ce que** celle-ci est configurée sous la forme d'une lentille intraoculaire ou d'une lentille de contact ou d'un verre de lunette.

10. Procédé de modification d'un pouvoir de réfraction d'une lentille ophtalmique, qui comprend une composition ophtalmologique selon l'une quelconque des revendications 1 à 7 et/ou qui est configurée selon l'une quelconque des revendications 8 ou 9, selon lequel ledit au moins un composé commutateur moléculaire est commuté entre un premier et un deuxième état au moyen d'un stimulus, le pouvoir de réfraction de la lentille ophtalmologique étant ainsi modifié, ledit au moins un premier élément structural et le deuxième élément structural correspondant n'étant mis en réaction intramoléculairement l'un avec l'autre qu'à un des états.
